# EUROPÄISCHE PATENTSCHRIFT

(11) **EP 2 677 939 B1**
(45) Veröffentlichungstag und Bekanntmachung des Hinweises auf die Patenterteilung: **15.07.2015**
(21) Anmeldenummer: 12705125.8
(22) Anmeldetag: 17.02.2012
(51) Int. Cl.: A61F 2/38, A61B 17/16, A61B 17/56, A61B 17/15, A61F 2/46

(54) **PATIENTENSPEZIFISCHER PROBEREPOSITIONSBLOCK**
PATIENT SPECIFIC TRIAL REPOSITIONING BLOCK
BLOC DE RÉDUCTION D'ESSAI SPÉCIFIQUE AU PATIENT

(30) Priorität: 21.02.2011 CH 301112011
(43) Veröffentlichungstag der Anmeldung: 01.01.2014
(73) Patentinhaber: MicroPort Orthopedics, Inc., Arlington, TN 38002 (US)
(72) Erfinder: Weber, Christoph, 5643 Sins (CH)
(74) Vertreter: Rentsch Partner AG
(86) Internationale Anmeldenummer: PCT/EP2012/052814
(87) Internationale Veröffentlichungsnummer: WO 2012/113735

(56) Entgegenhaltungen:
- EP-A2- 2 042 111
- WO-A1-95/17129
- WO-A2-2006/135728
- US-A1- 2003 093 079
- US-A1- 2009 087 276

## Beschreibung

### TECHNISCHES GEBIET

Die vorliegende Erfindung bezieht sich auf das Gebiet der Endoprothetik, insbesondere der totalen Knie-Arthroplastik. Sie betrifft einen Probe-Repositionsblock gemäss Anspruchs 1, sowie ein Herstellungsverfahren gemäss Anspruch 14.

### STAND DER TECHNIK

Heutige Instrumente und Operationstechniken für die Implantation einer Knie-Endoprothese sind alle nach dem selben Prinzip aufgebaut, d.h. die Prothese wird möglichst anatomisch anhand von knöchernen Landmarken positioniert.

Dies geschieht durch das Positionieren von Schnittblöcken, resp. Sägeführungen durch verschiedene Instrumente, welche die Bandverhältnisse im Knie berücksichtigen oder nicht.

Durch diese Schnittblöcke werden die verschiedenen Knochenschnitte geführt und präzise mit einer oszillierenden Säge reseziert. Dabei gibt es grundsätzlich zwei Philosophien bei der Knie-Arthroplastik: i) "Femur first", das heisst knochenreferenziert oder ii) "Tibia first", das heisst weichteilreferenziert. Bereits in der EP 0322363 A1 ist beschrieben, dass die Verwirklichung der richtigen mechanischen Beinachse eine der wichtigsten Voraussetzungen für eine lange Lebensdauer einer Knieprothese ohne Lockerung und Schmerz ist. Dabei kommt es nicht nur darauf an, den richtigen statischen Knieaufbau (alignment) herzustellen, sondern es ist ebenfalls wichtig, dass die ausgewogene Spannung der Kniebänder, soweit sie erhalten bleiben bzw. die richtige Weichteilspannung vorliegt. (F.C. Ewald: Biomechanical Indications for Implant Selection III: Knee. Role of Ligaments and Alignment, American Academy of Orthopaedic Surgeons, 54th Annual Meeting, San Francisco, January 22 -27, 1987. Instructural Course Number 323).

Die heute üblichen primären Totalknieprothesen bestehen aus einer am Femur und einer an der Tibia befestigten Komponente, die im Gegensatz zur Scharnierprothese nicht durch künstliche mechanische Hilfsmittel miteinander verbunden sind. Wie beim natürlichen Kniegelenk wird die flexible Verbindung zwischen den beiden Komponenten durch Bänder und Muskeln hergestellt, soweit diese bei der Knie-Arthroplastik, d.h. bei der Resektion und anschliessenden Implantation der Prothese erhalten bleiben.

Bevor die Prothesenkomponenten auf dem Femur bzw. der Tibia durch Verklemmen, Verkeilen und/oder Knochenzement befestigt werden können, müssen Femur und Tibia mit Hilfe von Knochensägen und anderen Instrumenten in die zur Prothese passende Form gebracht werden. Die im allgemeinen von den Herstellern der Knieprothese angebotenen dazu verwendeten Instrumente dienen in erster Linie dazu, die hierfür erforderlichen korrespondierenden Knochenschnitte am Femur und an der Tibia, die sogenannten Osteotomien mit der notwendigen Genauigkeit vorzunehmen.

Dabei ist es eine wesentliche Forderung, dass die bei der Biegung und Streckung des Knies aneinandergleitenden Komponenten der Knieprothese immer die anatomisch richtige Stellung zueinander haben, d.h. dass die mechanische Beinachse maximal 3° Varus oder 3° Valgus von der physiologischen Beinachse abweichen darf und dass eine Equilibrierung des Bandapparates erreicht wird, die sowohl bei der Extension als auch bei der Flexion eine gute Stabilität des Kniegelenks bewirkt. Ziel ist es jeweils ein gut ausbalanciertes künstliches Gelenk zu erhalten (Biege/Streckspalt-Verhältnis und varus/valgus Balance in Biegung und Streckung), welches in der HKA- (hip, knee, ankle) Achse, das heisst die mechanische Beinachse korrekt ausgerichtet ist. Ebenso soll eine volle Streckung sowie eine möglichst hohe Biegung des künstlichen Kniegelenkes ermöglicht werden.

Die bekannten Operationsmethoden versuchen dies wie folgt zu erreichen:

### i) Femur first (knochenreferenziert):

Zuerst wird der Femur anhand von knöcherenen Landmarken fertig bearbeitet. Anschliessend wird die Tibia reseziert und fertig bearbeitet. Erst bei der Probereposition mit den Probeprothesen sind die tatsächlichen Bandverhältnisse für den Operateur/Chirurgen ersichtlich.

Es ist bei dieser Operationstechnik möglich, aber nicht üblich, die Gelenk-Spaltmasse bei Biegung und Streckung zu messen. Jedoch erst nach dem distalen Schnitt am Femur. Die Femur-Rotation wird mit den meisten Systemen anhand der dorsalen Femur Condylen referenziert und sollte parallel zur Epycondylarachse oder zur sogenannten "Whiteside line" sein.

### ii) Tibia first (weichteilreferenziert):

Die Tibia wird zuerst reseziert, und anschliessend werden die Schnittblöcke am Femur anhand von knöchernen Landmarken, aber unter Berücksichtigung der Bandverhältnisse positioniert (Femur-Rotation). Das heisst, dass die letztendliche Positionierung der Femurprothese nicht immer den knöchernen Landmarken, z. B. Epycondylarachse entspricht, dafür aber erreicht werden kann, das sie absolut ausbalanciert implantiert ist.

Die Schnittblöcke werden bei beiden Operationstechniken über extra- oder intramedulläre Ausrichtinstrumente, welche anhand von Knochenlandmarken ausgerichtet werden, positioniert. Auch bei der Computer navigierten Operationstechnik (CAS) werden die Schnittblöcke anhand derselben Knochenlandmarken ausgerichtet. Der Vorteil ist, dass man die Landmarken, Beinachsen usw. und die Schnittblöcke visualisiert auf dem Bildschirm vor sich hat. Die Schnittblöcke können nun, mit Sensoren versehen, welche von einer Kamera erfasst werden, ohne zusätzliche Instrumente positioniert werden.

Fazit: Bei allen Techniken müssen die knöchernen Landmarken intraoperativ definiert werden, was durch Weichteile usw. zum Teil erschwert ist und vom Chirurgen ein hohes Mass an Konzentration, Fähigkeiten und Erfahrung erfordert, da die Dauer der Operation möglichst kurz gehalten werden sollte.

### PATIENTENSPEZIFISCHE SCHNITTBLÖCKE

Seit einiger Zeit bieten nun verschiedene Hersteller patientenspezifische Schnittblöcke an. Von dem zu behandelnden Knie wird vor der Operation eine Computertomographie- (CT-) oder Kernspin- (MRT-) Aufnahme angefertigt um ein genaues dreidimensionales (3D) Abbild zu erzeugen und die Beinachsen zu erfassen. Die 3D-Daten werden an den Prothesenhersteller geschickt, welcher die Prothese und die entsprechenden Schnittblöcke basierend auf dem exakten 3D-Modell des schadhaften Gelenks planen kann. Der Arzt kann die fertige Planung online bearbeiten und anschliessend seine Zustimmung zur Herstellung der Schnittblöcke ausgeben. Ebenso kann er Standardparameter angeben, wie zum Beispiel Achsangaben, Tibia-Slope, Femur-Rotation usw.

Danach erhält er die Schnittblöcke oder Pin-Blöcke (ohne Sägeführung, nur zur Positionierung der Pins) inkl. 3D Modellen der proximalen Bereiche der Tibia und des distalen Endes des Femurs. Fakt ist, dass mit den patientenspezifischen Schnittblöcken zwar dank des 3D Modells wesentlich exakter, aber im Prinzip genauso wie bei den bereits oben beschrieben unspezifischen Techniken, anhand der knöchernen Landmarken die Prothese resp. die Knochenschnitte geplant und ausgeführt werden.

Die Bandverhältnisse können bei der Planung nicht berücksichtigt werden. Intraoperativ können die Bandverhältnisse nur wie oben unter "Femur first" Technik beschrieben, berücksichtigt werden.

Grundsätzlich werden bei den bekannten Techniken ein Femur-Implantat am distalen Ende des Femurs befestigt, respektive ersetzt es diesen und ein tibiales Implantat, das eine Grundplatte zur Befestigung an der Tibia und eine artikulare Oberfläche aufweist, ersetzt das proximale Ende der Tibia. Mit einem Patella-Rückflächenersatz kann auch das Femur-Patella- Gelenk versorgt werden. Für die Befestigung des Femur-Implantats reseziert der Chirurg das distale Ende des Femurs derart, dass es passgenau komplementär zur inneren Oberfläche des femoralen Implantats zugeschnitten ist, um dieses formschlüssig aufzunehmen. Bei der "Femur-first" Operationsmethode rezesiert der Chirurg anschliessend das proximale Ende der gegenüberliegenden Tibia mit dem sogenannten Tibiaschnitt, um die Fläche zur Aufnahme der komplementären Grundplatte zu schaffen. Das femorale Implantat und die Tibia-Grundplatte werden beispielsweise mit Haftmitteln oder Befestigern an den jeweiligen Knochen befestigt. Die artikulare Oberfläche wird an der dem femoralen Implantat zugewandten Seite der Tibia-Grundplatte befestigt. Nachdem die Patella und das umgebende weiche Gewebe, einschliesslich der Bänder, Sehnen und Muskeln, wieder korrekt um das Knie herum angeordnet sind, ist das femorale Implantat in der Lage, auf der Gelenkoberfläche in einer Weise abzuwinkeln, die einer natürlichen Kniebewegung nahe kommt.

Patientenspezifische Schnittblöcke sind zum Beispiel aus der WO 2010/099142 und US 2009087276 bekannt.

Bei den vorgängig beschriebenen patientenspezifischen Schnittblöcken ist es bisher nicht möglich die Bandverhältnisse in Streckung und/oder Biegung oder die HKA-Achse vor dem distalen Femurschnitt zu kontrollieren. Nur eine nachträgliche Korrektur ist möglich und bringt unnötigen Knochenverlust und eine verlängerte OP Zeit mit sich.

### DARSTELLUNG DER ERFINDUNG

Es ist daher eine Aufgabe der Erfindung, eine patientenspezifische Vorrichtung zur Verfügung zu stellen, die es erlaubt mit einfachsten Mitteln die Weichteil- und Bandverhältnisse intraoperativ zu berücksichtigen, die Zeitdauer der Operation zu verringern und gleichzeitig deren Qualität und damit schlussendlich die Lebensdauer der Knie-Arthroplastik zu erhöhen.

Es ist weiterhin eine Aufgabe der Erfindung, ein Verfahren zur Herstellung eines patientenspezifischen Proberepositionsblocks anzugeben, das zumindest wesentliche Nachteile der bekannten Verfahren nicht aufweist.

Diese und andere Aufgaben werden durch die Merkmale des Probe-Repositionsblocks gemäss Anspruch 1 und das Herstellungsverfahren gemäss Anspruch 14 gelöst.

Die im folgenden beschriebene Lösung resp. Erfindung mit dem patientenspezifischen Probe-Repositionsblock bereichert die bekannte Operationstechnik mit patientenspezifischen Schnittblöcken dadurch, dass die Weichteilsituation, resp. Bandspannungsverhältnisse intraoperativ überprüft und korrigiert werden können. Es ermöglicht einen chirurgischen Eingriff in einer Genauigkeit und Exaktheit die bisher dank eines patientenspezifischen Schnittblockes nur in der knochenreferenzierten Operationstechnik zur Verfügung stand. Die vorliegende Erfindung entspricht beiden angewandten Philosophien: "Tibia first" und "Femur first".

### Beschreibung im Detail

Die patientenspezifischen Schnittblöcke bieten erhebliche Kostenvorteile. Sie bringen bis zu 30 Min. Zeitersparnis (1 OP Minute = ca. CHF 80.-, 5 Siebe weniger sterilisieren = CHF 100.-pro Sieb) was die Mehrkosten für einen CT Scan mehrfach einspart. Die verringerte Operationsdauer wirkt sich aber nicht nur positiv auf die Kosten der Operation aus, sondern sie verringert auch ganz wesentlich das Infektionsrisiko für den Patienten, da das Knie viel weniger lange geöffnet sein muss. Weitere Vorteile des neuen erfindungsgemässen Probe-Repositionsblocks liegen darin, dass weniger Instrumente bei der Operation eingesetzt werden müssen und sich dadurch der Schulungsbedarf für Ärzte und OP Personal reduziert.

Die vorgängig erstellten CT Scans oder MRIs erlauben bei dieser Operationsmethode, auch beim Arthrose-geschädigten Knie eine hochgenaue Implantation nach intakten anatomischen Landmarken. Die patientenspezifischen Schnittblöcke bieten daher die unbestritten genauste Positionierung der Knie-Endoprothese, besser als CAS und alle anderen bei dieser Operation bisher gebräuchlichen und bekannten Techniken.

Das Patientenrisiko wird zudem durch folgende Vorteile reduziert, respektive der Operationserfolg wird gesteigert:
- kein Eröffnen des Intramedulärkanals nötig und damit unter anderem ein wesentlich verringertes Risiko für eine Fettembolie
- verringerte OP Zeit (da weniger Schritte und weniger massiver Eingriff) und dadurch eine geringere Infektionsgefahr
- genaueste Planung und Positionierung inklusive genauester Grössenbestimmung der Implantat-Komponeneten
- Möglichkeit, die mechanischen Achsen von Tibia und Femur konventionell (zum Beispiel mit einem Ausrichtstab) zu kontrollieren

Folgende Nachteile der bekannten knochenreferenzierten Operationstechniken mit patientenspezifischen Schnittblöcken werden vermieden:
- Keine Möglichkeit die Bandverhältnisse in Streckung und/oder Biegung vor dem distalen Femurschnitt zu kontrollieren. Nur eine nachträgliche Korrektur ist möglich, was zu einer verlängerten OP Zeit und unnötigem Knochenverlust führt.
- Femurrotationskorrekturen sind über konventionelle Instrumente erst nach dem distalen Schnitt möglich, was wiederum die OP Zeit verlängert.
- Der Operateur sieht erst nach dem Tibiaschnitt und dem distalen Femurschnitt die tatsächlichen Bandspannungsverhältnisse in Extension. Erst nach dem Positionieren des anterioren/posterioren Schnittblockes auf dem distalen Schnitt lassen sich die Bandspannungsverhältnisse in Flexion prüfen.

Der patientenspezifische Probe-Repositionsblock gemäss der vorliegenden Erfindung vereinigt gemäss bevorzugter Ausführungsformen tatsächlich die Funktionalität eines Proberepositions-, Korrektur- und Schnittblocks. Dabei bietet er Korrekturmöglichkeiten bereits vor dem ersten Femurschnitt. Er stellt sicher, dass dem Operateur die sicherste, genauste, schnellste und kostengünstigste Implantationsmethode einer Knie-Endoprothese zur Verfügung steht.

Mittels des neuen patientenspezifischen Femur-Proberepositions-, Korrektur- und Schnittblocks, der im Folgenden der Einfachheit halber nur noch Probe-Repositionsblock genannt wird, kann bereits nach dem Tibiaschnitt eine Probereposition durchgeführt werden. So kann sich der Operateur bereits nach dem Tibiaschnitt ein sehr genaues Bild über das Endresultat der Operation machen. Dies war bisher nicht möglich.

Ermöglicht wird dies nun dadurch, dass die Aussenmasse des erfindungsgemässen patientenspezifischen Probe-Repositionsblocks genau definiert sind. Das heisst der Tibia- udn der

Femur-Aufbau sind im Block einberechnet, welcher so dimensioniert ist, dass er während des Einsatzes bei der Operation den Femur in der geplanten "korrigierten" End-Position auf der Tibia zu liegen bringt.

Die Planung einer Knie-Endoprothese am 3D Modell nach CT Scan und nach knöchernen Landmarken, ist unbestritten die genaueste Methode ein Implantat zu positionieren. Nur hatte der Operateur bei dieser genauen aber knochenreferenzierten Methode bisher keinerlei Informationen bezüglich der Bandverhältnisse, bevor er den Femur (distalen Femurschnitt) bearbeitet hatte.

Die Rotationskorrektur des Femurs konnte ebenfalls erst nach dem distalen Femurschnitt mittels Augenmass durch Rotieren des 4in1 Blockes erreicht werden.

Gemäss der vorliegenden Erfindung ist es nun möglich die genaue Planung am 3D Modell bereits nach dem ersten, ebenfalls am CT Modell geplanten, mittels vorzugsweise patientenspezifischem Tibia-Schnittblock, reseziertem Tibiaschnitt zu überprüfen.

Dem Operateur gibt dies die Möglichkeit die Weichteile, resp. die Bandspannung zu kontrollieren und wenn nötig anzupassen, bevor er den Femur bearbeitet. Der Operateur kann, wenn nötig, ein Band- und Kapselrelease vornehmen um das Gelenk auszubalancieren. Ebenfalls kann er eine Biege- und Streckspaltmessung vornehmen und die Gesamtbeinachse überprüfen. Dies ist direkt mit Hilfe des Probe-Repositionsblocks, und allenfalls mit Hilfe von Spacern (Spaltmesslehre) oder mit einem gängigen Knee-Analyzer resp. Drucksensoren möglich. Letztere lassen sich gemäss bevorzugter Ausführungsformen in den Probe-Repositionsblock gemäss der Erfindung integrieren oder in geeignete Aufnahmebereiche des Probe-Repositionsblock gemäss weiterer Ausführungsformen lösbar befestigen.

Genau so, können zum Beispiel mittels Spacern unterschiedliche Inlay-Höhen (Meniskus PE-Teil) und unterschiedliche Femurgrössen (Biegespalt zu gross oder zu klein) simuliert werden. Wenn dann trotz dem Release knöcherne Korrekturen bezüglich varus/valgus Beinachse und oder Femur-Rotation nötig sind, können diese direkt mit Hilfe des neuen Probe-Repositionsblocks gemacht werden, da dieser vorzugsweise eine Anzahl von Paaren von Korrektur-Pinbohrlöchern aufweist, die das Setzen von Pins für korrigierte Achsen ermöglichen. Diese Pin-Bohrlöcher sind derart am Probe-Repositionsblock angeordnet, dass sie genau definierte Alternativpositionen für Pins bei einer allfällig nötigen Verschiebung der "Joint Line" oder eine varus-/ valgus- Korrektur (für den distalen Femur-Schnitt) oder für die Rotation oder für den anterior/posterior (a/p) shift der Femurkomponente (Referenzlöcher für Pins zur Befestigung des 4in1-Schnittblocks) bieten.

Gemäss weiterer bevorzugter Ausführungsformen der vorliegenden Erfindung umfasst der Repositionsblock einsetzbare Einsätze, mittels derer die geplante oder vorgegebene diskrete Korrekturpositionen der Pin-Bohrlöcher ausgewählt werden können. Gemäss weiterer vorteilhafter Ausführungsformen kann die Korrekturposition der Pin-Bohrlöcher nach Bedarf am Korrektureinsatz eingestellt werden.

Im folgenden wird ein kurzer Überblick zur Operations-Technik gemäss der vorliegenden Erfindung gegeben:
- Kniegelenk eröffnen, Osteophyten entfernen
- Tibia mit vorzugsweise patientenspezifischem Tibia-Schnittbock nach CT Planung resezieren. Eventuell kann zur Sicherheit an der Tibia auch weniger reseziert werden.
- Schutzblech auf Tibiaschnitt legen, distalen Teil des Blockes gemäss der Erfindung auf Femur aufsetzen, Knie in Streckung bringen, Streckspaltmessung durchführen und HKA Achse prüfen
- Knie flektieren, patientenspezifischer Probe-Repositionsblock auf Femur aufsetzen (dorsaler Blockteil), Patella einspuren und Biegespaltmessung vornehmen.
- Wenn alles in Ordnung ist -> distaler Femurschnitt, Referenzlöcher für 4 in 1 Schnittblock bohren, Tibia und Femur endbearbeiten, Probereposition, Implantation Korrekturmöglichkeiten

### Weichteil- und Bandrelease, dorsales Release (Femur)

Wenn das Weichteilrelease nicht ausreicht, können die Korrekturpositonen definierenden Korrektur-Pin-Bohrlöcher genutzt werden um damit zum Beispiel "Joint Line"-Verschiebungen und varus-/valgus-Korrekturen vorzunehmen, respektive die Bohrungen für die Pins für einen konventionellen distalen Schnittblock entsprechend der gewünschten Korrektur zu setzen.

Die distalen Referenzlöcher für den 4 in 1 Block können in verschiedenen Rotations- und a/p-Positionen durch den Block gebohrt werden. Je nach Kniesystem ist es möglich auch die Femurgrösse zu ändern (anterior oder posterior referenzierte Referenzlöcher).

### Beschreibung der Schnittblöcke

Der Tibia-Schnittblock bleibt unverändert (wie von verschiedenen Herstellern angeboten)

Es ist ein wesentliches Merkmal der vorliegenden Erfindung, dass der Probe-Repositionsblock für den Femur patientenspezifisch hergestellt ist. Unter patientenspezifisch wird nicht nur die spezifische Anpassung einer proximalen Oberfläche des Probe-Repositionsblocks an die Oberfläche des distalen Femurbereichs oder zumindest an wesentliche Landmarken innerhalb dieses Bereichs verstanden, sondern dass der Probe-Repositionsblock zudem in Hinblick auf den distalen und den dorsalen Femur-Prothesen- und Tibia-Aufbau genau definiert dimensioniert ist.

Insbesondere die Lage und die Position der distalen und der dorsalen Seitenwand des Probe-Repositionsblocks sind in Bezug auf die proximale Oberfläche des Probe-Repositionsblocks oder zumindest in Bezug auf wesentliche Landmarken-Referenzbereiche innerhalb dieser proximalen Oberfläche genau vorgegeben.

Der Probe-Repositionsblock gemäss der vorliegenden Erfindung definiert mit seiner distalen Seitenwand eine distale Referenzfläche, die im eingesetzten Zustand gemäss Operationsplanung beim gestreckten Knie, das heisst im Extensionszustand vorzugsweise exakt auf dem Tibiaschnitt, respektive auf dem Schutzblech auf dem Tibiaschnitt zu liegen kommt. Gemäss weiterer Ausführungsformen ist die distale Seitenwand schwächer dimensioniert, so dass die distale Referenzfläche nicht direkt auf dem Tibiaschnitt, respektive auf dem Schutzblech auf dem Tibiaschnitt zu liegen kommt, sondern auf einem zusätzlich eingesetzten Spacer von bekannter Dicke.

Distal und dorsal kann der Block konvexe Kondylen aufweisen, ebenso medial und lateral.

Mit seiner dorsalen Seitenwand definiert der Probe-Repositionsblock gemäss der vorliegenden Erfindung eine dorsale Referenzfläche, die im eingesetzten Zustand gemäss Operationsplanung beim gebeugten Knie, das heisst im Zustand der Flexion vorzugsweise exakt auf dem Tibiaschnitt, respektive auf dem Schutzblech auf dem Tibiaschnitt zu liegen kommt. Auch hier kann wiederum die Wandstärke reduziert und ein Spacer definierter Dicke eingesetzt werden.

In Bezug auf die distale und dorsale Oberfläche des Probe-Repositionsblocks ist die proximale Oberfläche des Probe-Repositionsblocks patientenspezifisch hergestellt. Die distale und die dorsale Referenz-Oberfläche der zugehörigen Seitenwände definieren, von den jeweiligen Schnitten aus gemessen, den distalen respektive den dorsalen Femuraufbau der Femurkomponente, sowie den gesamten Tibiaaufbau.

Die Dicke eines Schutzbleches zum Schutz des Tibiaschnittes kann berücksichtigt werden und bei der Dimensionierung des Probe-Repositionsblocks in Abzug gebracht werden.

Die distale Referenz-Oberfläche ist derart angeordnet, dass sie die Gelenkstellung im gestreckten Zustand vorgibt.

Für den Fachmann ist basierend auf der vorliegenden Lehre verständlich, dass die Referenz-Oberflächen nicht von der gesamten Seitenwand gebildet werden müssen, sondern dass die tatsächliche Ausdehnung der ebenen Referenzfläche auf einen kleineren Bereich im Schnittpunkt der jeweiligen Bezugsachse beschränkt sein kann. Im Extremfall kann auch die gesamte Fläche einen Radius aufweisen. Bevorzugt wird jedoch mit einer diskreten ebenen Referenzfläche gearbeitet.

Die distale und die dorsale Referenz-Oberfläche des Probe-Repositionsblocks definieren einen gemeinsamen Referenz-Winkel. Gemäss bevorzugter Ausführungsformen entspricht dieser Referenzwinkel dem Beugewinkel, der von der anatomischen Femurachse und der anatomischen Tibiaachse im gebeugten Zustand bei der Beugespaltmessung gebildet wird (0°/90°).

Ein Probe-Repositonsblock zum Definieren von mindestens einer Schneidebene auf einem Femur bei einer totalen Knie-Arthroplastik gemäss der vorliegenden Erfindung, umfasst mindestens einen patientenspezifischen proximalen Referenzbereich, der gegen die distale Oberfläche des Femurs gelegt wird und eine distale Seitenwand, die eine distale Referenzfläche definiert , welche gemäss der patientenspezifischen Operationsplanung bei der Extension des Kniegelenks mit dem Tibiaschnitt zusammenfällt, und eine dorsale Seitenwand, die eine dorsale Referenzfläche definiert welche gemäss der patientenspezifischen Operationsplanung bei der Flexion des Kniegelenks mit dem Tibiaschnitt zusammenfällt.

Gemäss einer bevorzugten Ausführungsform des Probe-Repositonsblocks sind die distale und/oder die dorsale Referenzfläche jeweils direkt von der Oberfläche der jeweiligen distalen und/oder dorsalen Seitenwand gebildet.

Gemäss einer weiteren vorteilhaften Ausführungsform sind die distale und/oder die dorsale Referenzfläche von Spacern gebildet, die an den jeweiligen Seitenwänden angeordnet werden, wobei die Spacer vorzugsweise lösbar an den Seitenwänden befestigbar sind.

Der erfindungsgemässe Probe-Repositonsblock ist vorteilhafterweise dadurch gekennzeichnet, dass das anteriore Blockteil eine Schneidführung für einen distalen Femurschnitt umfasst, die ein Werkzeug zum Erstellen des distalen Femurschnitts führen kann, wobei die Schneidführung eine Referenzebene für den distalen Femurschnitt definiert, die derart zur distalen Referenzebene des Probe-Repositionsblocks ausgerichtet ist, dass diese beiden Ebenen dem Extensionsspalt gemäss der patientenspezifischen Operationsplanung entsprechen.

Das Mass des Streckspalts wird also direkt von der Dicke der distalen Seitenwand simuliert, oder es wird - gemäss weiterer Ausführungsformen - zusätzlich gezielt eine kleinere Dicke gewählt und das Mass unter Einsatz eines Spacers (d.h. einer Spaltmesslehre) eingestellt. Genauso wie die Streckspaltmessung lässt sich auch die Biegespaltmessung mit Spacer durchführen.

Der Einsatz von Spacern hat den zusätzlichen Vorteil, dass man verschiedene Inlay-Dicken durch verschiedene Spacerdicken sehr einfach simulieren kann. Ebenfalls kann das Verhältnis respektive der Unterschied zwischen dem Biege und dem Streckspalt gemessen werden.

Der Probe-Repositonsblock gemäss der Erfindung umfasst vorzugsweise ein anteriores und ein dorsales Blockteil, welche lösbar in genau definierter Stellung zueinander aneinander befestigbar sind. Der zweiteilige Aufbau mit dem abnehmbarem dorsalen Blockteil hat sich als besonders vorteilhaft bei der Streckspalt und Beinachsenmessung (HKA Achse) erwiesen, da der dorsale Teil des Blockes diese Messung durch Umlenken der dorsalen Kapsel beeinflussen würde und bei der Biegespaltmessung, da der distale Teil des Blockes die Messung mit eingespurter Patella verhindern würde.

Der dorsale Blockteil umschliesst vorzugsweise die dorsalen Kondylen des Femurs für die Biegespaltmessung bei eingespurter Patella. Die entsprechende Oberfläche des dorsalen Blockteils ist wiederum patientenspezifisch anhand der 3D Daten gefertigt, die zur Grössenbestimmung bei der Operationsplanung im CT oder MRI ohnehin erfasst werden.

Der Probe-Repositionsblock muss passgenau auf der Gelenksfläche (Trochlea) aufliegen (nötig, damit die Osteophyten vorgängig entfernt werden können und die Bandspannungen nicht durch "umlenken" beeinflusst werden) und die äussersten Punkte der distalen und dorsalen Kondylen umschliessen.

Die distale und dorsale Oberfläche ist vorzugsweise glatt gestaltet.

Beim Probe-Repositonsblock gemäss der vorliegenden Erfindung sind das dorsale Blockteil an der dorsalen Seitenwand und das distale Blockteil an der distalen Seitenwand vorzugsweise mit Aussparungen zur Aufnahme von Sensoren und/oder direkt mit Sensoren versehen.. Die Sensoren sind vorzugsweise Drucksensoren und besonders bevorzugt medial und lateral an der jeweiligen Seitenwand angeordnet. Die Sensoren sind entweder bereits ab Werk direkt integriert, oder es sind Aussparungen oder Aufnahmen vorhanden, so dass in der Klinik auf Wunsch oder bei Bedarf entsprechende Sensoren montiert werden können.

Bei definiertem kleinerem Mass: distal und dorsal sind die Flächen mit medialen und lateralen konvexen Rundungen (Kondylen) versehen. Diese Kondylen sind abnehmbar in unterschiedlichen Dicken erhältlich um verschiedene Korrekturen zu simulieren. (Resektionsmenge, varus/valgus, Femur-Rotation, Inlay-Dicken, Femur-Grössen). Ebenfalls können diese mit Sensoren ausgestattet sein. Vorzugsweise wird die Messung dann mit medialen oder lateralen Spacern durchgeführt, um die oben genannten Korrekturen zu simulieren.

Gemäss weiterer vorteilhafter Ausführungsformen umfasst der Probe-Repositionsblock neben der distalen Schnittführung alle weiteren Femur-Schnittführungen. Das anteriore Blockteil weist also zusätzlich eine Schneidführung für einen zweiten (zum Beispiel einen posterioren) Femurschnitt und/oder einen dritten (zum Beispiel einen anterioren) Femurschnitt und/oder allfällige weitere Femurschnitte auf.

Vorzugsweise ist eine anteriore Seitenwand mit Positionierungmittel, besonders bevorzugt in Form von verschiedenen Körrektur-Pin-Bohrloch-Paaren (+2mm, +4mm, 0mm, -2mm, -4mm; 1°- 3° varus/valgus) versehen um einen konventionellen distalen Schnittblock bei Bedarf distalisieren, proximalisieren, varisieren oder valgisieren zu können. Von den jeweils zusammengehörenden Bohrlöchern eines Paares ist vorzugsweise je eines im medialen und eines im lateralen Bereich der Seitenwand angeordnet um über einen möglichst grossen Abstand eine hohe Präzision der definierten Ebene zu erzielen.

In weiteren vorteilhaften Ausführungsformen sind an der distalen Seitenwand verschiedene Bohrlöcher vorhanden (1° in/out 2° in/out 3° in/out 5° in/out 7°/ +2mm, - 2mm), anhand derer die Rotation und a/p Position des 4in1 Blockes (a/p Schnitte, resp. Femurkomponente) eingestellt werden kann. Von den jeweils zusammengehörenden Bohrlöchern eines Paares ist vorzugsweise wiederum je eines im medialen und eines im lateralen Bereich der Seitenwand angeordnet.

Der erfindungsgemässe Block kann vorzugsweise derart gestaltet sein, dass er Schnittblöcke aus konventionellen Instrumenten direkt aufnehmen kann, wie es zum Beispiel aus der WO 2010/099142 bekannt ist.

Der erfindungsgemässe Probe-Repositionsblock umfasst gemäss weiterer vorteilhafter Ausführungsformen Aufnahmen für extramedulläre Achskontrollinstrumente, wie sie aus dem Stand der Technik bekannt und im Markt erhältlich sind.

Gemäss weiterer erfindungsgemässen Ausführungsformen sind im erfindungsgemässen Block eine oder mehrere Aufnahmen von einer/mehrerer Referenz/en für verschiedene CAS Systeme vorgesehen.

Der Probe-Repositionsblock gemäss der vorliegenden Erfindung ist vorzugsweise ganz in einem Rapid-Prototyping-Verfahren hergestellt. Es sind zumindest die patientenspezifischen Anteile, insbesondere der patientenspezifische proximale Referenzbereich und vorzugsweise die dorsale und die distale Seitenwand der dorsalen und anterioren Blockteile in einem Rapid-Prototyping-Verfahren hergestellt. Unter Rapid-Prototyping-Verfahren werden Herstellugnsverfahren verstanden, bei denen Bauteile anhand von 3D Daten direkt, vorzugsweise schichtweise aus formlosen oder formneutralen Materialien aufgebaut werden. Es kommen derzeit insbesondere 3D Printing Verfahren in Frage bei denen Kunststoffe, Kalkpulver mit Epoxid-Hülle oder Photopolymere auf Acylbasis verwendet werden, oder Elektronenstrahlschmelz-Verfahren für Metalle, oder Fused Deposition Modeling (FDM)-Verfahren für Acrylnitril-Butadien-Styrol-Copolymerisate (ABS) oder Polycarbonate, oder Laminated Object Modelling (LOM)-Verfahren für Papier, Kunststoffe, Keramik oder Aluminium, oder Laser Engineered Net Shaping (LENS)-Verfahren für Metalle, oder Laserauftragschweißen für Metalle oder Selektives Laserschmelzen (SLM) für Metalle, Kunststoffe oder Keramiken, oder Selektives Lasersintern (SLS) für Thermoplaste (zum Beispiel: Polycarbonate, Polyamide, Polyvinylchlorid, Metalle, Keramiken) oder Stereolithografie (STL oder SLA)-Verfahren für flüssige Duromere oder Elastomere.

Die vorliegende Erfindung realisiert folgende Vorteile gegenüber bereits erhältlichen patientenspezifischen Schnittblöcken. Sie wird den beiden Operations-Philosophien "knochenreferenziert" und "weichteilreferenziert" gerecht, indem sie die Vorteile beider Techniken vereint. Sie erlaubt es bereits nach dem Tibiaschnitt das Endresultat gemäss der patientenspezifischen Operationsplanung zu beurteilen und bei Bedarf die geeigneten Korrekturen vorzunehmen. Es kann zudem ein Band- und Kapsel-Release vorgenommen werden, bevor der Femur bearbeitet wird. Die ligamentären Verhältnisse sind die selben wie mit der Probeprothese oder der definitiven Prothese.

Bei den Fällen, bei denen die ligamentären Verhältnisse, die nun frühzeitig kontrollierbar sind, im Zusammenhang mit den geplanten Schnitten in Ordnung sind, kann enorm viel Zeit eingespart werden. Bei den Fällen, bei denen Korrekturen notwendig sind, basieren diese Korrekturen direkt auf der sehr exakten patientenspezifischen Planung, die wiederum auf 3D Daten eines CT- oder MRI-Scan beruhen.

So lässt sich zum Beispiel eine 2° varus Korrektur für den distalen Schnitt am Femur, die exakt einer Abweichung der mechanischen Femurachse von 2° entspricht, direkt und exakt durch den neuen Probe-Repositionsblock mit Hilfe der Positionierungmittel, zum Beispiel in Form von Pin-Bohrloch-Paaren, vornehmen.

Beim neuen Verfahren zum Herstellen eines patientenspezifischen Proberepositionsblocks gemäss der Erfindung zum Definieren von mindestens einer Schneidebene auf einem Femur bei einer totalen Knie-Arthroplastik, ist es wesentlich, dass basierend auf 3D-Daten des zu behandelnden Knies mindestens ein patientenspezifischer proximaler Referenzbereich, der gegen die distale Oberfläche des Femurs F gelegt wird, hergestellt wird und eine distale Seitenwand derart hergestellt wird, dass sie eine distale Referenzfläche definiert, welche gemäss der patientenspezifischen Operationsplanung bei der Extension des Kniegelenks mit dem Tibiaschnitt zusammenfällt. Vorzugsweise wird zudem eine dorsale Seitenwand derart hergestellt, dass sie eine dorsale Referenzfläche definiert, welche gemäss der patientenspezifischen Operationsplanung bei der Flexion des Kniegelenks mit dem Tibiaschnitt zusammenfällt.

Die Seitenwände müssen dabei nicht durchgehend flächig ausgebildet sein, sondern mindestens drei Auflagepunkte umfassen, die in der jeweiligen Referenzebene liegen und diese definieren. Vorzugsweise sind zwei seitliche Auflagebereiche ausgebildet, die die Referenzebene definieren.

### KURZE ERLÄUTERUNG DER FIGUREN

Die Erfindung soll nachfolgend anhand von Ausführungsbeispielen im Zusammenhang mit der Zeichnung näher erläutert werden.
- Fig. 1a: zeigt eine schematische Darstellung des menschlichen Beinskeletts im präoperativen und
- Fig. 1b: im postoperativen Zustand, wobei jeweils ein linkes Bein mit den relevanten Achsen dargestellt ist.
- Fig. 2a: zeigt einen erfindungsgemässen Probe-Repositionsblock im zusammengesetzten Zustand in einer Ansicht auf die distale Seite; und

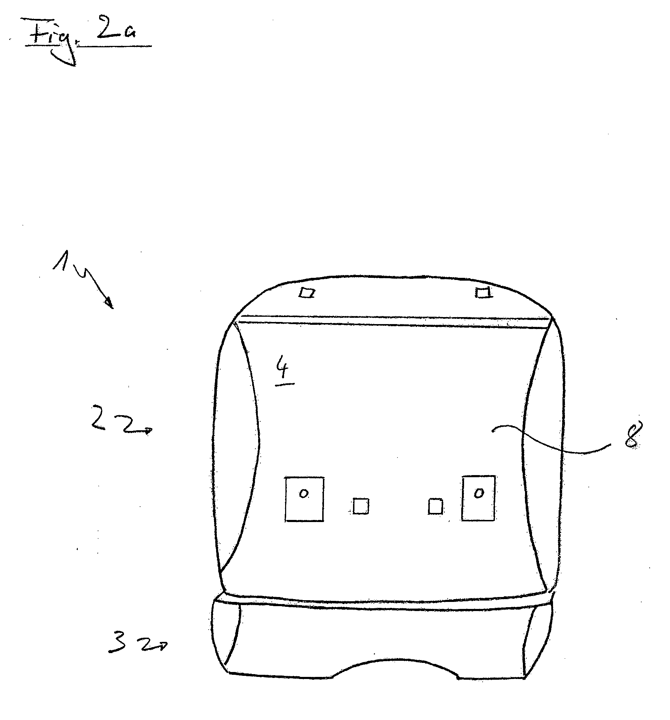

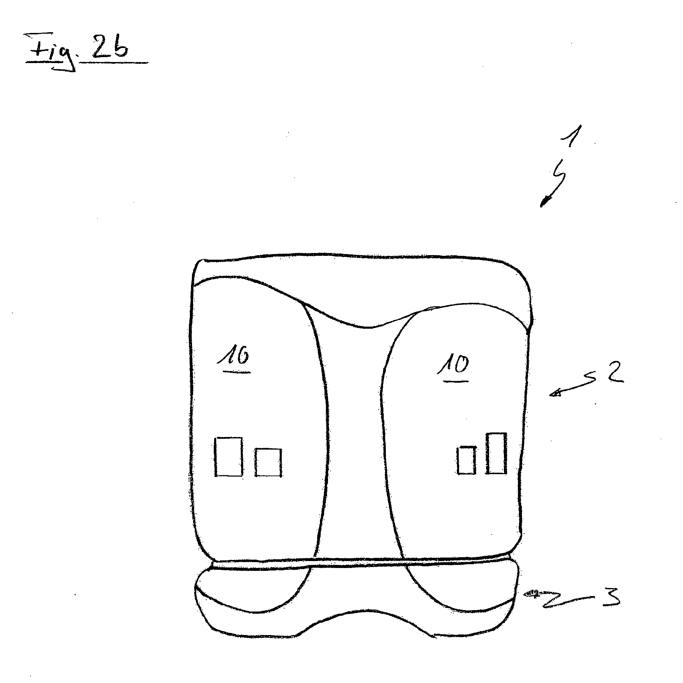

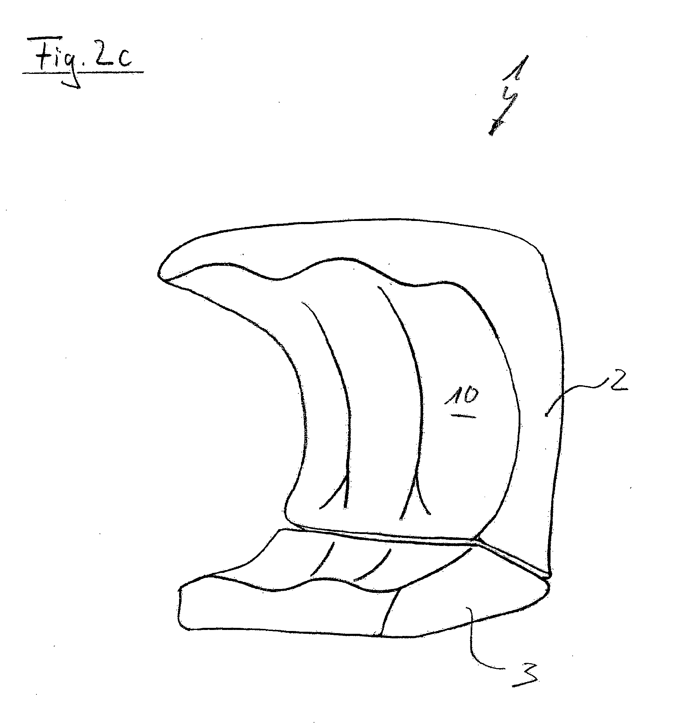

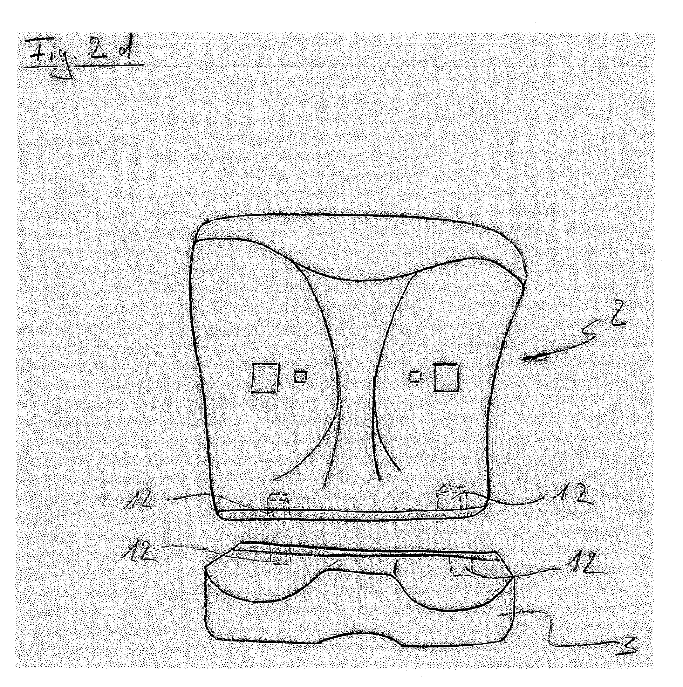

- Fig. 2: b eine Ansicht auf die proximale Seite des Probe-Repositionsblocks gemäss Figur 2a.
- Fig. 2c: zeigt eine perspektivische Ansicht schräg auf die proximale Seite gemäss Figur 2b, und
- Fig. 2d: den Probe-Repositionsblock gemäss der Figuren 2a - 2c, wobei die beiden Untereinheiten des Probe-Repositionsblocks voneinander getrennt in einer Ansicht gemäss Figur 2b dargestellt sind.

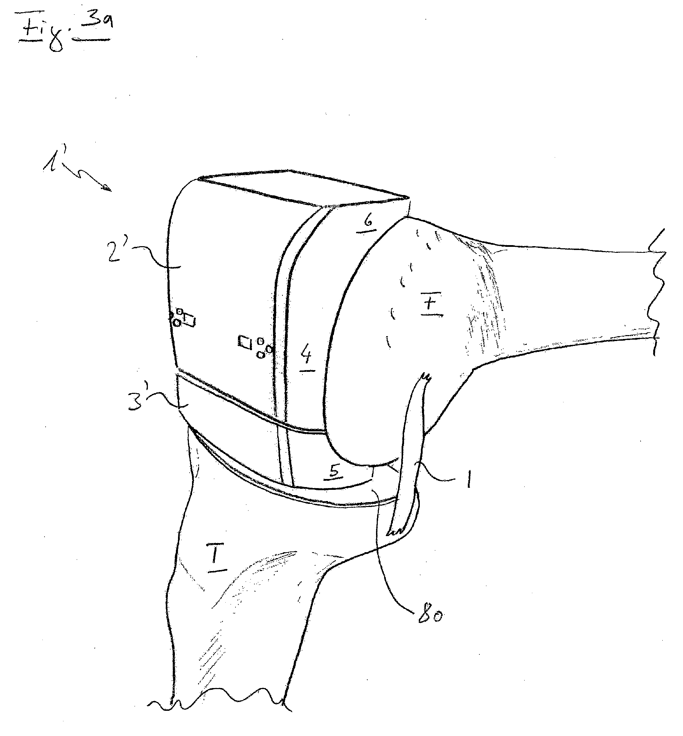

- Fig. 3a: zeigt in einer schematischen Ansicht in einer perspektivischen Ansicht von ventro-lateral ein Kniegelenk in gebeugtem Zustand (Flexion) bei dem nach bereits erfolgtem Tibiaschnitt der Probe-Repositionsblock zur Kontrolle des Flexionsspalts am Femur angeordnet ist, und

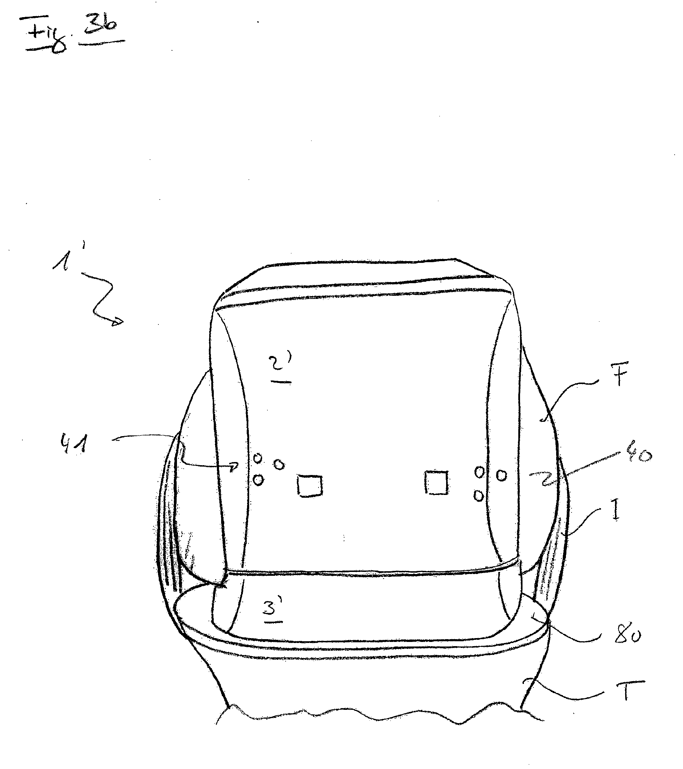

- Fig. 3b: eine direkte Ansicht auf die distale Seite des Probe-Repositionsblocks im gebeugten Knie gemäss der Figur 3a.

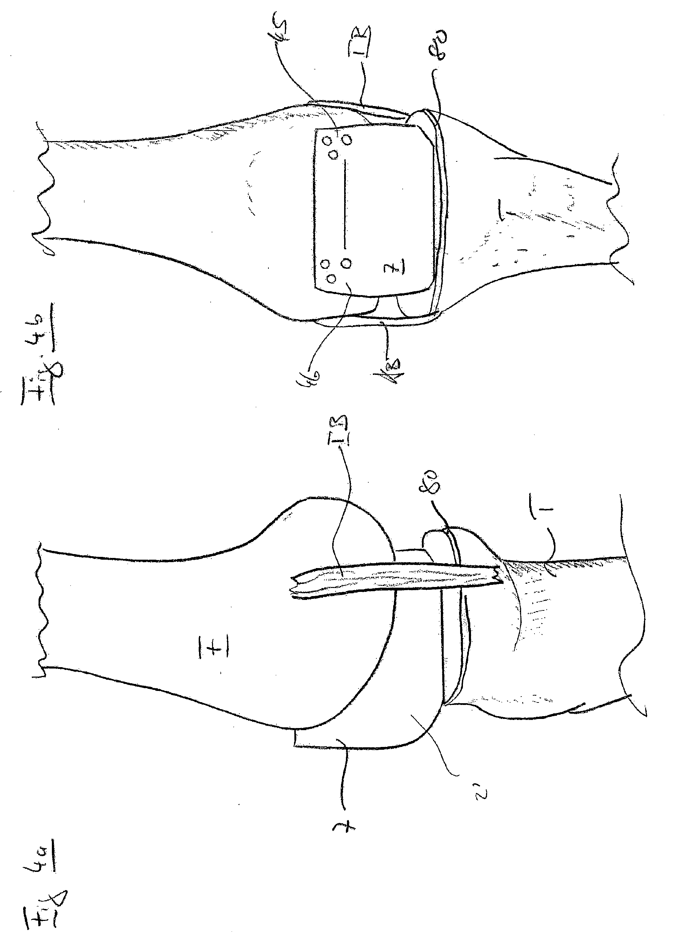

- Fig. 4a: laterale Ansicht eines Kniegelenks in gestrecktem Zustand (Extension) bei dem nach erfolgtem Tibiaschnitt der Probe-Repositionsblock zur Kontrolle des Extensionsspalts am Femur angeordnet ist, wobei der dorsale Teil des Probe-Repositionsblocks entfernt ist.
- Fig. 4b: zeigt die Situation gemäss Figur 4a in einer Ansicht von Ventral.

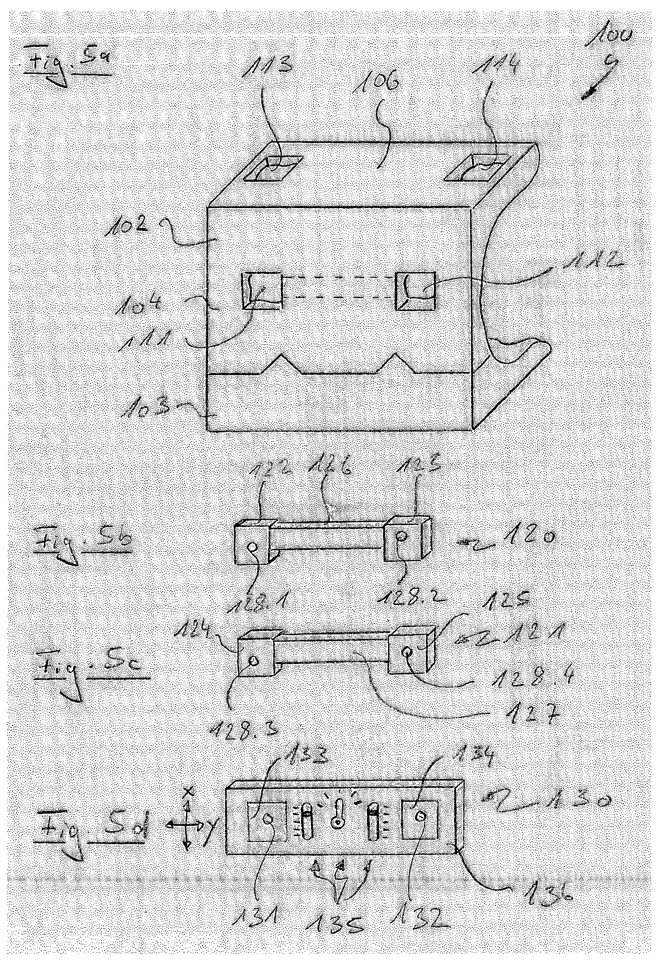

- Fig. 5a: zeigt einen Probe-Repositionsblock gemäss einer weiteren Ausführungsform der Erfindung zur Verwendung mit Korrektureinsätzen die in der Figur fehlen.
- Fig. 5b: zeigt einen Korrektureinsatz zur Verwendung mit einem Probe-Repositionsblock gemäss Figur 5a.
- Fig. 5c: zeigt einen weiteren Korrektureinsatz zur Verwendung mit einem Probe-Repositionsblock gemäss Figur 5a.
- Fig. 5d: zeigt einen einstellbaren Korrektureinsatz zur Verwendung mit einem Probe-Repositionsblock gemäss Figur 5a

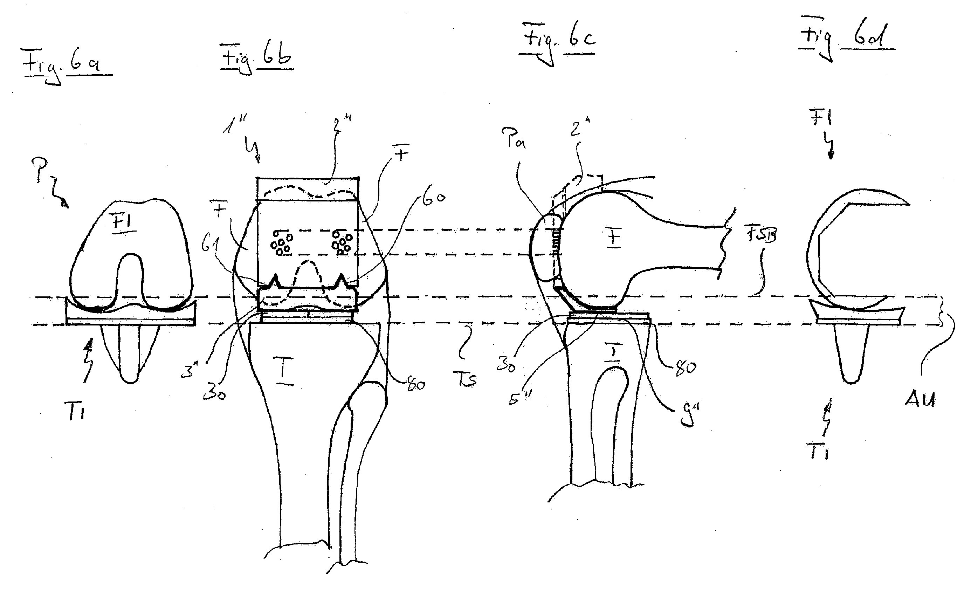

- Fig. 6: zeigt eine schematische Übersicht in der beim gebeugten Knie in
- Fig. 6a: eine Sicht auf die distale Seite eines Femur-Implantats auf einem TibiaImplantat, in
- Fig. 6b: eine Ansicht auf die distale Seite eines am Femur angeordneten Probe-Repositionsblocks mit Schutzblech und zusätzlichem Spacer auf der Tibia, in
- Fig. 6c: eine laterale Ansicht auf die Situation gemäss der Figur 6b und in
- Fig. 6d: eine laterale Ansicht auf die Implantate gemäss Fig. 6a dargestellt ist, wobei alle Ansichten hinsichtlich der Tibiaschnittebene zueinander ausgerichtet sind.

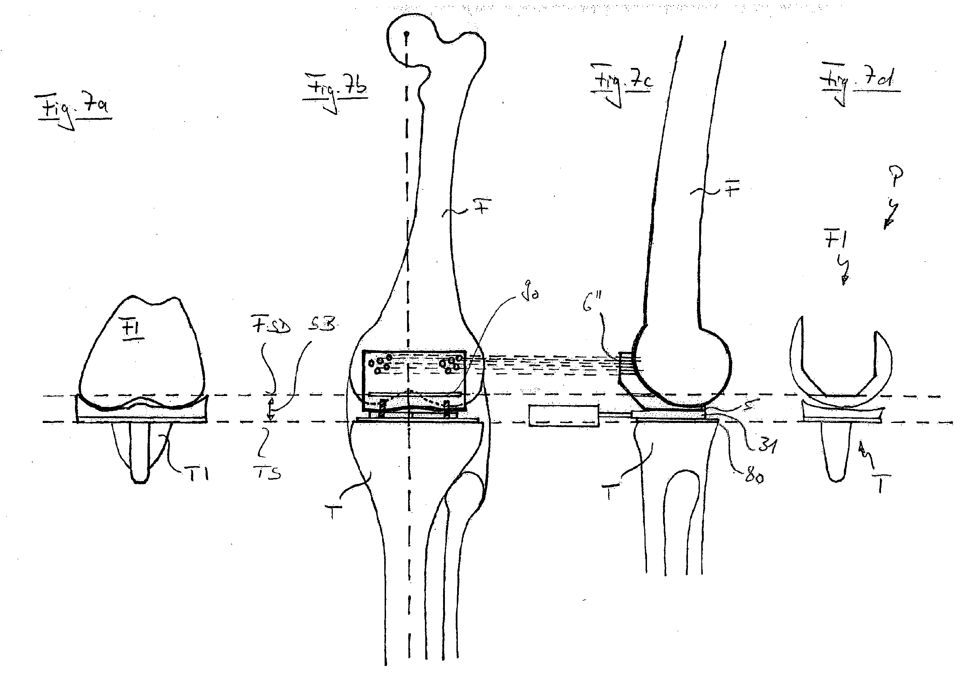

- Fig. 7: zeigt eine schematische Übersicht in der beim gestreckten Knie in
- Fig. 7a: eine Sicht auf die ventrale Seite eines Femur-Implantats das auf einem TibiaImplantat angeordnet ist, in
- Fig. 7b: eine Ansicht auf die distale Seite eines am Femur angeordneten Probe-Repositionsblocks gemäss einer weiteren Ausführungsform mit Schutzblech auf der Tibia, in
- Fig. 7c: eine laterale Ansicht auf die Situation gemäss der Figur 7b und in
- Fig. 7d: eine laterale Ansicht auf die Implantate gemäss Fig. 7a dargestellt ist, wobei alle Ansichten hinsichtlich der Tibiaschnittebene zueinander ausgerichtet sind.

### WEGE ZUR AUSFÜHRUNG DER ERFINDUNG

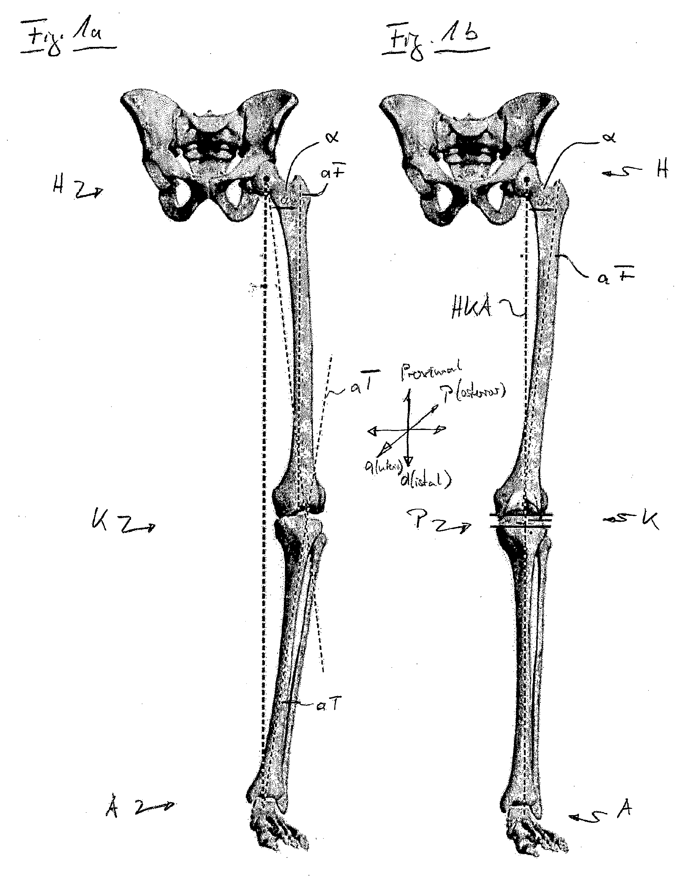

In den Figuren 1a und 1b ist in einer schematischen Darstellung das menschliche Beinskeletts im präoperativen (Fig. 1a) und im postoperativen Zustand
mit eingesetzter primärer Totalknieprothese (Fig. 1 b) dargestellt, wobei jeweils ein linkes Bein mit den anatomischen Achsen von Tibia aT und Femur aF, der HKA-Achse und dem Valguswinkel α gezeigt ist.

Anhand der in der Figur 2 dargestellten verschiedenen Ansichten eines erfindungsgemässen Probe-Repositionsblocks 1 gemäss einer ersten Ausführungsform der vorliegenden Erfindung soll im folgenden auf einige wichtige Aspekte der vorliegenden Erfindung eingegangen werden. In der Figur 2a ist der Probe-Repositionsblock 1 im zusammengesetzten Zustand in einer Ansicht auf die distale Seite gezeigt. Der Probe-Repositionsblock 1 setzt sich aus einem anterioren Blockteil 2 und einem dorsalen Blockteil 3 zusammen. Die Befestigungsmittel, die die beiden Blockteile form- und/oder kraftschlüssig in der vorgesehenen Position zusammenhalten, sind in der Figur nicht dargestellt. Eine distale Seitenwand 4 definiert eine im wesentlichen ebene distale Referenzfläche 8, welche gemäss der patientenspezifischen Operationsplanung bei der Extension des Kniegelenks mit dem Tibiaschnitt zusammenfällt, respektive bei der Probereposition auf der geschnittenen Tibiaoberfläche oder auf einem darauf angeordneten Schutzblech zu liegen kommt.

In der Figur 2b ist eine Ansicht auf die proximale, patientenspezifisch geformte Seite des Probe-Repositionsblocks 1 gemäss der Figur 2a dargestellt.

Aus der perspektivischen Ansicht gemäss der Figur 2c wird nochmals die patientenspezifische Gestaltung der proximalen Seite, respektive des proximalen Referenzbereichs 10 des Probe-Repositionsblocks 1 deutlich. Die proximalen Anteile des anterioren Blockteils 2 sowie auch des posterioren Blockteils 3 sind genau an die Oberfläche des zu ersetzenden Femurs des Patienten angepasst. Die patientenspezifische Ausgestaltung der proximalen Referenzbereiche ist beim dargestellten Ausführungsbeispiel annächernd vollflächig auf die vorgängig ermittelten 3D Daten des zu ersetzenden Gelenkbereichs angepasst, so dass eine hochgenaue Positionierung des Blocks 1 am Femur/ den Femurkondylen sichergestellt ist. Das dargestellte Implantat ist auch bezüglich der Dicke der distalen Seitenwand 4 und der dorsalen Seitenwand 5 individuell auf den Patienten, respektive auf die patientenspezifische Operationsplanung angepasst. Ausgehend von den geplanten Gelenk-Spaltmassen der Operationsplanung ist die Wanddicke 40 der distalen Seitenwand 4 im dargestellten Ausführungsbeispiel so gewählt, dass die von der distalen Seitenwand 4 definierte distale Referenzfläche 8 (in Zusammenschau mit der Figur 2a) den gewünschten Streckspalt von zum Beispiel 18 mm vorgibt.

Der Block 1 gemäss der Figuren 2a bis 2d umfasst selbst keine Schneidführung für einen distalen und allenfalls weitere Femurschnitte, die ein Werkzeug zum Erstellen dieser Femurschnitte führen kann. Sie weist aber Positionierungmittel in Form von verschiedenen Pin-Bohrloch-Paaren auf, mittels derer Bohrungen zur Aufnahme von Pins zur Positionierung eines konventionellen Schnittblock am Femur positionsgenau erstellt werden können. Von den jeweils zusammengehörenden Bohrlöchern eines Paares für die Rotationskorrektur ist je eines im medialen und eines im lateralen Bereich der Seitenwand 4 angeordnet um über einen möglichst grossen Abstand eine hohe Präzision der definierten Ebene zu erzielen.

In der distalen Seitenwand 4 des Blocks 1 sind, wie in den Figuren 2a, 2b und 2d angedeutet, gemäss bevorzugter Ausführungen Sensoren 70 integriert oder integrierbar, die zur Belastungsmessung bei der Extension dienen. Die Sensoren, wie auch die Pin-Bohrloch-Paare sind in den Figuren 2a - 2d nicht in allen Ansichten dargestellt.

In der Figur 2d sind das anteriore Blockteil 2 und das posteriore Blockteil 3 voneinander beanstandet dargestellt. Aus der Zusammenschau mit der Figur 2c wird deutlich, dass gemäss bevorzugter Ausführungsformen die relative Positionierung der beiden Blockteile zueinander nicht nur von Formschlussmitteln bewirkt werden kann, wie es im folgenden noch gezeigt wird, sondern auch von Kraftschlussmitteln wie zum Beispiel Magneten 12, die gegenpolig aufeinander ausgerichtet in den Blockteilen versenkt sind. In der Figur 2d sind diese Magnete 12 strichliniert angedeutet.

In der Figur 3a ist schematisch dargestellt, wie ein zweiteiliger Probe-Repositionsblock 1' gemäss einer Ausführungsform der Erfindung die im wesentlichen dem Probe-Repositionsblock 1 gemäss der Figur 2 entspricht, im Einsatz an einem gebeugten Knie angeordnet ist. In der Figur sind der besseren Übersichtlichkeit halber alle Weichteile weggelassen und nur einen Innenband I ist schematisch angedeutet. Eine dorsale Seitenwand 5', des dorsalen Blockteils 3' definiert eine dorsale Referenzfläche, die im eingesetzten Zustand gemäss Operationsplanung beim gebeugten Knie, das heisst im dargestellten Zustand der Flexion exakt auf dem Tibiaschnitt, respektive auf dem Schutzblech 80 auf dem Tibiaschnitt zu liegen kommt. Die Wandstärke der dorsalen Seitenwand 5' ist exakt auf die patientenspezifische Operationsplanung angepasst und definiert eine dorsale Referenzfläche, die bei der Flexion des Kniegelenks mit dem Tibiaschnitt zusammenfällt, respektive bei der Probereposition auf einem auf dem Tibiaschnitt angeordneten Schutzblech 80 zu liegen kommt.

In der Figur 3b ist die Situation gemäss der Figur 3a in einer Aussicht auf die distalen Seitenflächen der beiden Blockteile 2' und 3' dargestellt. Der behandelnde Chirurg kann mit Hilfe des patientenspezifischen Probe-Repositionsblocks 1 den Beugespalt, wie er gemäss der Operationsplanung erstellt wurde, direkt prüfen. Stellt er bei dieser Prüfung fest, dass die Dimensionierung des Beugespalts und/oder die vorgesehene Rotation nicht auf die Patienten spezifische Weichteilssituation passt, so kann er mit Hilfe der Korrektur-Pin-Bohrlöcher diesen Fehler sofort korrigieren und die Bohrungen für die Aufnahme der Pins im Femur abweichend von der Operationsplanung derart anbringen, dass das Gelenk in der Flexion optimal weichteilreferenziert ausbalanciert ist.

In den Figuren 4a und 4b wird beim Kniegelenk gemäss der Figur 3a und 3b mit Hilfe des anterioren Blockteils 2' die Probereposition beim gestreckten Knie vorgenommen. Aus der Figur 4a wird deutlich, dass der posteriore Blockteil 3' zur Überprüfung der Operationsplanung in der Extension entfernt ist. Es kommt nur noch das anteriore Blockteils 2' zum Einsatz um eine allfällig nötige Verschiebung der "Joint Line" oder eine varus-/ valgus- Korrektur (für den distalen Femur-Schnitt) vorzunehmen. Der anteriore Blockteil 2' liegt mit seiner distalen Seitenwand 4 direkt auf dem Schutzblech 80 auf dem Tibiaschnitt auf. Im dargestellten Ausführungsbeispiel bildet also die distale Wandfläche der distalen Seitenwand 4 direkt die distale Referenzfläche.

Stellt der Chirurg fest, dass der Streckspalt gemäss der Operationsplanung nicht optimal auf die Weichteilsituation des Patienten abgestimmt ist, oder dass die angestrebten Varus- und Valguswinkel nicht erreicht werden, so kann er wiederum mittels der in der frontalen Seintewand 7 angeordneten Korrektur-Pin-Bohrlöcher 45, 46 die Bohrungen für die Pins unter Berücksichtigung der notwendigen Korrekturen setzen.

In der Figur 5a ist ein Probe-Repositionsblock 100 gemäss einer weiteren Ausführungsform der vorliegenden Erfindung dargestellt. Der Probe-Repositionsblock 100 ist wiederum in ein anteriores Blockteil 102 und ein posteriores Blockteil 103 unterteilt. Die distale Seitenwand 104 und die ventrale Seitenwand 106 des anterioren Blockteils 102 sind jeweils mit zwei Aufnahmeöffnungen 111, 112, 113 und 114 versehen. In die Aufnahmeöffnungen können verschiedene Korrektureinsätze 120, 121, wie sie zum Beispiel in der Figur 5b und der Figur 5c gezeigt sind, eingesetzt werden. Die Aufnahmeöffnungen 111-114 weissen einen definierten quadratischen Querschnitt auf und sind so dimensioniert, dass die Korrektureinsätze 120, 121 von der proximalen Seite her eingeführt werden können. Die Korrektureinsätze umfassen jeweils zwei laterale Einschubkörper 122, 123, 124 und 125 die über einen Tragbalken 126, 127 miteinander verbunden sind. Die Einschubkörper 122, 123, 124 und 125 die Vertikalbalken 126, 127 sind so dimensioniert, dass sie im eingesetzten Zustand von Proximal so weit in die jeweilige Seitenwand eingeschoben werden können, dass keine Anteile in den proximalen Referenzbereich ragen und den Formschluss mit der Femuroberfläche stören. Jeder der Einschubkörper 122, 123, 124 und 125 weist ein Pin-Bohrloch 128.1 - 128.4 auf.

Der in der Figur 5b dargestellte Korrektureinsatz 120 wird in die Aufnahmeöffnungen 113,114 der ventralen Seitenwand 106 eingeschoben und definiert im eingesetzten Zustand mit seinem Paar von Pin-Bohrlöchern 128.1 und 128.2 die gemäss Operationsplanung vorgesehene Position der zwei anterioren Pins, an denen anschliessend ein konventioneller Schnittblock in der geplanten distalen/proximalen Position mit 0mm und 0° Varus/Valgus-Abweichung gemäss Operationsplanung befestigbar ist. Stellt der Chirurg bei der Probereposition am gestreckten Knie einen Korrekturbedarf hinsichtlich Varus oder Valgus oder hinsichtlich einer distalen oder proximalen Abweichung der Schnittebene des geplanten distalen Femurschnitts fest, so kann er mittels einer Reihe von vorgegebenen Korrektureinsätzen genau definierte Korrekturen vornehmen. Die Korrektureinsätze bieten vorzugsweise folgende Korrekturmöglichkeiten und Kombinationen davon an:
Distal/proximal: +1mm, +2mm, +3mm, +4mm, -1 mm, -2mm, -3mm, -4mm
Varus (jeweils links/rechts): 1 °, 2°, 3°, 4°
Valgus (jeweils links/rechts): 1 °, 2°, 3°, 4°

Der in der Figur 5c dargestellte Korrektureinsatz 121 wird in die Aufnahmeöffnungen 111,111 der ventralen Seitenwand 104 eingeschoben und definiert im eingesetzten Zustand mit seinem Paar von Pin-Bohrlöchern 128.3 und 128.4 die gemäss Operationsplanung vorgesehene Position der zwei distalen Pins, an denen ein konventioneller Schnittblock in der geplanten Position mit 0mm Abweichung von den anterioren/posterioren Schnittebenen und mit 0° Abweichung von der geplanten Innen/Aussen-Rotation befestigbar ist. Stellt der Chirurg bei der Probereposition am gebeugten Knie einen Korrekturbedarf hinsichtlich Rotation oder hinsichtlich einer anterioren oder posterioren Abweichung der Schnittebene des geplanten dorsalen Femurschnitts fest, so kann er wiederum mittels einer Reihe von vorgegebenen Korrektureinsätzen genau definierte Korrekturen vornehmen. Die Korrektureinsätze bieten vorzugsweise folgende Korrekturmöglichkeiten und Kombinationen davon an:
Anterior/posterior: +2mm, -2mm
Aussenrotation: -1 °, -2°, -3°
Innenrotation: 1°, 2°, 3°

Gemäss weiterer nicht in den Figuren dargestellten Ausführungsformen sind die Korrektureinsätze derart ausgebildet, dass sie nicht wie die in Figuren 5b und 5c dargestellten Einsätze 120, 121 von proximal in die entsprechenden Aufnahmeöffnungen eingeschoben, sondern von der jeweils anderen Seite der Wand her. Die Einsätze weisen dazu auf der Rückseite Form- und/oder Kraftschlussmittel auf, so dass diese Korrektureinsätze lösbar am Blockteil befestigbar sind. Der Vorteil dieser Ausgestaltung liegt darin, dass die Korrektureinsätze einfach austauschen lassen, auch wenn das Blockteil am Femur angebracht ist.

In der Figur 5d ist ein Korrektureinsatz 130 gemäss einer weiteren bevorzugten Ausführungsform dargestellt, bei dem die Position der beiden Pin-Bohrlöcher 131 und 132 im Korrektureinsatz in x- und y-Richtung veränderbar ist. Die Pin-Bohrlöcher 131 und 132 sind dazu jeweils auf einer innerhalb des Korrektureinsatzes verschiebbeweglich gelagerten Tragplatte 133, 134 angeordnet, die mittels der Verstelleinrichtungen 135 in eine gewünschte x-y-Position gebracht werden können und gemäss der vorgenannten Korrekturwerte Varus/Valgus, distal/proximal respektive Innen/Aussen-Rotation, anterior/posterior eingestellt werden können. Der in der Figur 5d dargestellte Korrektureinsatz 130 wird wiederum nicht wie die in Figuren 2b und 2c dargestellten Einsätze 120, 121 von proximal in die entsprechenden Aufnahmeöffnungen eingeschoben, sondern von der jeweils anderen Seite der Wand her. Seine Grundplatte 136 weist dazu auf der, der Rückwand, die in der Zeichungsansicht hinten liegt Form- und/oder Kraftschlussmittel auf, so dass der Korrektureinsatz 130 lösbar am Blockteil 103 befestigbar ist.

In den Übersichtsdarstellungen gemäss der Figuren 6a bis 6d wird der Zusammenhang zwischen der Dimensionierung des erfindungsgemässen patientenspezifischen Probe-Repositionsblocks 1" und der vorgängig erstellten Operationsplanung und dem einzusetzenden Implantaten nochmals verdeutlicht.

In der Figur 6a ist eine Totalknieprothese umfassend ein Femurimplantat FI und ein Tibiaimplantat TI in einer Ansicht von Ventral im quasi gebeugten Zustand dargestellt. In der Figur 6d ist die selbe Totalknieprothese in einer lateralen Ansicht gezeigt. Die einzusetzende Totalknieprothese wird im Rahmen der Operationsplanung passend für den Patienten ausgewählt. In der Figur 6 sind zwei für die Operationsplanung wichtige Ebenen, die von der Prothese vorgegeben werden, strichliniert dargestellt. Es sind dies die Ebene für den Tibiaschnitt TS und die Ebene für den posterioren Femurschnitt FSP. In der Figur beträgt der dargestellte Abstand zwischen diesen beiden Ebenen zum Beispiel 18mm, was der Aufbauhöhe AU der ausgewählten Totalknieprothese entspricht.

Allgemein kann festgestellt werden, dass sich gemäss der vorliegenden Erfindung diese Aufbauhöhe im gebeugten und die Aufbauhöhe im gestreckten Zustand bereits nach dem Ausführen des Tibiaschnitts durch die Patientenspezifischen Proberepositionsblöcke simulieren lässt, da die jeweilige dorsale oder distale Seitenwand in der Dicke derart dimensioniert ist, dass sie in Bezug auf den jeweiligen dorsalen respektive distalen Femurschnitt die geplante Aufbauhöhe vorgibt, so dass der Femur bereits während der Probereposition in den patientenspezifisch geplanten Positionen zu liegen kommt. Diese Positonen lassen sich dadurch äusserst einfach hinsichtlich der korrekten Achsausrichtungen, Rotationen und Winkelstellungen und insbesondere auch hinsichtlich der korrekten Weichteilspannung prüfen und allenfalls korrigieren. Dies bereits nach Durchführung des Tibiaschnitts, ohne jedwede Invasion am Femur, das heisst, ohne dass am Femur ein Schnitt oder eine Bohrung angebracht worden ist.

In der Zusammenschau mit den Figuren 6b und 6c wird deutlich, dass diese beiden Referenzebenen die entscheidende Rolle für die Dimensionierung des patientenspezifischen Probe-Repositionsblocks 1", insbesondere für die Wandstärke der dorsalen Seitenwand 5" und die Positionierung der Pin-Bohrlöcher und der Korrektur-Pin-Bohrlöcher in der distalen Seitenwand 4" spielen.

Der in den Figuren 6b und 6c dargestellte Probe-Repositionsblocks 1" weist wiederum keine integrierte Schneidführung auf, sondern dient dazu mittels Pin-Bohrlöchern die Pins für einen konventionellen Schneidblock genau definiert setzen zu können. Beim Block 1" wird die dorsale Referenzfläche 9" nicht direkt von der dorsalen Seitenwand 5" gebildet, sondern von einem Spacer 30, der zwischen der dorsalen Seitenwand 5" und dem Schutzblech 80 liegt. Die Wandstärke der dorsalen Seitenwand 5" ist gemäss der dargestellten Ausführungsform um die Dicke des Spacers 30 verringert.

Aus der Ansicht gemäss der Figur 6c wird deutlich, dass die Trennlinie zwischen den beiden Blockteilen gemäss der vorliegenden Erfindung vorzugsweise derart gewählt ist, dass sich nach Entfernen des anterioren Blockteils die Patella mitsamt der Patellarsehen ungestört eingespuren lässt. Der posteriore Blockteil berührt die Patella im eingespurten Zustand nicht.

In der Ansicht gemäss der Figur 6b sind Formschlussmittel 60, 61 dargestellt, die zur korrekten Positionierung des anterioren Bolckteils 2" und dem posterioren Blockteil 3" zueinander dienen. Die Formschlussmittel sind im Bereich der Trennebene der beiden Blockteile als zwei lateral aufstehende Rippen 60, 61 auf dem posterioren Blockteil 3" ausgebildet, die in zwei korrespondierende Nuten 60, 61 im anterioren Blockteil 2" eingreifen.

In der Figur 7 ist in Analogie zur Figur 6 der Zusammenhang zwischen der Dimensionierung des erfindungsgemässen patientenspezifischen Probe-Repositionsblocks 1", insbesonders dessen distaler Seitenwand und der vorgängig erstellten Operationsplanung und dem einzusetzenden Implantaten nochmals verdeutlicht.

In der Figur 7a ist wiederum die Totalknieprothese P umfassend das Femurimplantat FI und das Tibiaimplantat TI in einer Ansicht von Ventral nun im quasi gestreckten Zustand dargestellt. In der Figur 7d ist die selbe Totalknieprothese P in einer lateralen Ansicht gezeigt. In der Figur 7 ist wiederum die für die Operationsplanung wichtige Tibia-Schnittebenen TS strichliniert eingezeichnet. In der Extension ist eine weitere von der Prothese P vorgegebenen Ebene hoch relevant, nämlich die Ebene für den distalen Femurschnitt FSD. In der Zusammenschau mit den Figuren 7b und 7c wird deutlich, dass diese beiden Referenzebenen die entscheidende Rolle für die Dimensionierung des patientenspezifischen Probe-Repositionsblocks 1", insbesondere für die Wandstärke der distalen Seitenwand 4" und die Positionierung der Pin-Bohrlöcher und der Korrektur-Pin-Bohrlöcher in der ventralen Seitenwand 6" spielen.

Im dargestellten Ausführungsbeispiel wird die distale Referenzfläche 8" nicht direkt von der distalen Seitenwand des anterioren Blockteils 2" gebildet, sondern von einer distalen Seitenwand eines Spacers in Form eines Knee-Analyzers 31, wie er aus dem Stand der Technik bekannt ist. Bei der Herstellung des erfindungsgemässen Probe-Repositionsblocks gemäss Figur 7 wird die Wandstärke der distalen Seitenwand um die Dicke des Knee-Analyzers 31 verringert.

Aus den Figuren 7a bis 7d wird deutlich, dass die Summe aus der Wandstärke der distalen Seitenwand des anterioren Blockteils 2", der Dicke des Spacers 31 und des Schutzblechs 80 genau der Breite des Streckspalts SB zwischen TibiaschnittTS und distalen Femurschnitt FSD, wie sie in der Operationsplanung vorgesehen ist, entspricht. Im dargestellten anterioren Blockteil 2" ist eine Schneidführung 90 zur Führung eines Schneidwerkzeugs zum Erstellen des distalen Femurschnitts genau in der Ebene des distalen Femurschnitts FSD angeordnet,

### Liste der Bezugszeichen

- A: Knöchel (ankle)
- AB: Aussenband
- aF: anatomische Femurachse
- aT: anatomische Tibiaachse
- C: Mechanische Beinachse
- D: Mechanische Femurachse
- E: Resektionstiefe Tibia (mm)
- F: Femur
- FSD: distaler Femurschnitt
- FSA: anteriorer/ventraler Femurschnitt
- FSB: dorsaler Femurschnitt
- FI: Femur-Implantat
- H: Hüfte
- IB: Innenband
- K: Knie
- P: Totalknieprothese
- Pa: Patella
- T: Tibia
- TI: Tibia-Implantat
- α: Valguswinkel

- 1: Probe-Repositionsblock
- 2: anteriores Blockteil
- 3: dorsales Blockteil
- 4: distale Seitenwand
- 5: dorsale Seitenwand
- 6: ventrale Seitenwand
- 7: frontale/ventrale Seitenwand
- 8: distale Referenzfläche
- 9: dorsale Referenzfläche
- 10: proximaler Referenzbereich
- 11: Referenzwinkel
- 12: Magnete
- 30: Spacer
- 31: Spacer, Knee-Analyzer
- 40: Korrektur-Pin-Bohrlöcher
- 41: Korrektur-Pin-Bohrlöcher
- 45: Korrektur-Pin-Bohrlöcher
- 46: Korrektur-Pin-Bohrlöcher
- 51: distale Seitenfläche
- 52: anteriore Seitenwand
- 53: anteriore Seitenfläche
- 60: Formschlussmittel
- 61: Formschlussmittel
- 70: Sensor
- 80: Schutzblech (Tibia-)
- 90: Schneidführung
- 100: Probe-Repositionsblock
- 102: anteriores Blockteil
- 103: dorsales Blockteil
- 104: distale Seitenwand
- 105: dorsale Seitenwand
- 106: ventrale Seitenwand
- 111 - 114: Aufnahmeöffnungen
- 120, 121: Korrektu rei nsätze
- 122 - 125: Einschubkörper
- 126, 127: Tragbalken
- 128.1-128.4: Pin-Bohrlöcher
- 130: Korrektureinsatz (verstellbar)
- 131, 132: Pin-Bohrlöcher
- 133, 134: Tragplatte
- 135: Verstelleinrichtungen
- 136: Grundplatte

## Patentansprüche

1. Probe-Repositonsblock (1, 1', 1", 100) zum Definieren von mindestens einer Schneidebene auf einem Femur F bei einer totalen Knie-Arthroplastik, umfassend:
- mindestens einen patientenspezifischen proximalen Referenzbereich (10), der gegen die distale Oberfläche des Femurs F gelegt wird,
- eine distale Seitenwand (4, 4', 4"),
- und/oder vorzugsweise eine dorsale Seitenwand (5, 5', 5"),
**dadurch gekennzeichnet, dass**
- die distale Seitenwand (4, 4', 4") eine distale Referenzfläche (8, 8', 8") definiert , welche gemäss der patientenspezifischen Operationsplanung bei der Extension des Kniegelenks mit dem Tibiaschnitt (TS) zusammenfällt,
- und/oder die dorsale Seitenwand (5, 5', 5") eine dorsale Referenzfläche (9, 9', 9") definiert, welche gemäss der patientenspezifischen Operationsplanung bei der Flexion des Kniegelenks mit dem Tibiaschnitt TS zusammenfällt,
wobei eine Dicke der distalen Seitenwand (4, 4', 4") und/oder der dorsalen Seitenwand (5, 5', 5") individuell auf den Patienten beziehungsweise die patientenspezifische Operationsplanung angepasst sind.

2. Probe-Repositonsblock (1) gemäss Patentanspruch 1, **dadurch gekennzeichnet, dass** die distale und/oder die dorsale Referenzfläche direkt von der Oberfläche der jeweiligen Seitenwand gebildet sind.

3. Probe-Repositonsblock (1) gemäss Patentanspruch 1, **dadurch gekennzeichnet, dass** die distale und/oder die dorsale Referenzfläche von Spacern (30) gebildet sind, die an den Seitenwänden angeordnet werden, wobei die Spacer (30) vorzugsweise lösbar an den Seitenwänden befestigbar sind.

4. Probe-Repositonsblock (1) gemäss einem der Patentansprüche 1 bis 3, **dadurch gekennzeichnet**,'dass der Probe-Repositionsblock ein anteriores (2) und ein dorsales (3) Blockteil umfasst, welche lösbar aneinander befestigbar und positionierbar sind.

5. Probe-Repositonsblock (1) gemäss Patentanspruch 4, **dadurch gekennzeichnet, dass** das anteriore Blockteil (2) eine Schneidführung für einen distalen Femurschnitt FSD oder Positionierungsmittel für einen Schneidblock für zumindest einen distalen Femurschnitt FSD umfasst, wobei die Schneidführung oder die Positonierungsmittel eine Referenzebene für den distalen Femurschnitt FSD definieren, die derart zur distalen Referenzebene des Probe-Repositionsblocks ausgerichtet ist, dass diese beiden Ebenen dem Extensionsspalt gemäss der patientenspezifischen Operationsplanung entsprechen.

6. Probe-Repositonsblock (1) gemäss Patentanspruch 5, **dadurch gekennzeichnet, dass** die anteriore Seitenwand mit Korrektur-Positionierungsmitteln versehen ist, besonders bevorzugt in Form von Korrektur-Pin-Bohrloch-Paaren oder Korrektureinsätzen um die Schneidführung für den ersten, distalen Femurschnitt FSD bei Bedarf distalisieren, proximalisieren, varisieren oder valgisieren zu können.

7. Probe-Repositonsblock (1) gemäss Patentanspruch 6, **dadurch gekennzeichnet, dass** die Positionierungsmittel für folgende Korrekturpositionen vorgesehen sind: +1, +2, +3, +4, 0, -1, -2, -3, -4; 1°, 2°, 3°, 4° varus/valgus um einen konventionellen distalen Schnittblock oder eine Schneidführung im Probe-Repositonsblock bei Bedarf distalisieren, proximalisieren, varisieren oder valgisieren zu können.

8. Probe-Repositonsblock (1) gemäss einem der Patentansprüche 5 bis 7, **dadurch gekennzeichnet, dass** das distale Blockteil mit Positionierungmittel versehen ist, vorzugsweise in Form von Pinloch Paaren für einen Schnittblock oder eine Schneidführung für einen dorsalen Femurschnitt FSB und anterioren Femurschnitt FSA, die eine Schneidführung/Werkzeug zum Erstellen des dorsalen Femurschnitts FSB und des anterioren Femurschnitts FSA führen kann, wobei die Positionierungmittel die Position der Schneidführung definieren, die wiederum eine Referenzebene für den Femurschnitt definiert, die derart zur dorsalen Referenzebene (9") des Probe-Repositionsblocks ausgerichtet ist, dass diese beiden Ebenen dem Flexionsspalt gemäss der patientenspezifischen Operationsplanung entsprechen.

9. Probe-Repositonsblock (1) gemäss Patentanspruch 8, **dadurch gekennzeichnet, dass** Korrektur-Positionierungsmittel für folgende Korrekturpositionen vorgesehen sind: 0° - 7° Innen- und Aussenrotation in 1° Schritten und +/- 1 - 4 mm anterior/posterior Shift.

10. Probe-Repositonsblock (1) gemäss einem der Patentansprüche 5 bis 9, **dadurch gekennzeichnet, dass** das anteriore Blockteil (2) eine Schneidführung für einen anterioren Femurschnitt FSA und/oder einen posterioren Femurschnitt FSB und/oder weitere Femurschnitte umfasst.

11. Probe-Repositonsblock (1) gemäss einem der Patentansprüche 4 bis 10, **dadurch gekennzeichnet, dass** das dorsale Blockteil (3) an der dorsalen Seitenwand (5) und das anteriore Blockteil (2) an der distalen Seitenwand (4) mit Aussparungen zur Aufnahme von Sensoren und/oder mit Sensoren versehen ist.

12. Probe-Repositonsblock (1, 100) gemäss einem der vorhergehenden Patentansprüche, **dadurch gekennzeichnet, dass** mindestens der patientenspezifische proximale Referenzbereich (10) und vorzugsweise die dorsalen und die distalen Seitenwände (3, 4) der dorsalen und anterioren Blockteile in einem Rapid-Prototyping-Verfahren hergestellt sind.

13. Probe-Repositonsblock (1, 100) gemäss einem der vorhergehenden Patentansprüche 4 bis 11, **dadurch gekennzeichnet, dass** das dorsale Blockteil mittels form- und oder kraftschlüssiger Mittel (12, 60, 61) miteinander lösbar verbindbar und in einer definierten Relativposition zueinander positionierbar sind.

14. Verfahren zum Herstellen eines patientenspezifischen Proberepositionsblocks (1, 1', 1", 100) zum Definieren von mindestens einer Schneidebene auf einem Femur F bei einer totalen Knie-Arthroplastik, wobei
- basierend auf 3D-Daten des zu behandelnden Knies mindestens ein patientenspezifischer proximaler Referenzbereich (10), der gegen die distale Oberfläche des Femurs F gelegt wird, hergestellt wird,
**dadurch gekennzeichnet, dass**
- eine distale Seitenwand (4, 4', 4") derart hergestellt wird, dass sie eine distale Referenzfläche (8, 8', 8") definiert , welche gemäss der patientenspezifischen Operationsplanung bei der Extension des Kniegelenks mit dem Tibiaschnitt (TS) zusammenfällt,
- und/oder eine dorsale Seitenwand (5, 5', 5") hergestellt wird, die eine dorsale Referenzfläche (9, 9', 9") definiert, welche gemäss der patientenspezifischen Operationsplanung bei der Flexion des Kniegelenks mit dem Tibiaschnitt TS zusammenfällt,
wobei eine Dicke der distalen Seitenwand (4, 4', 4") und/oder der dorsalen Seitenwand (5, 5', 5") individuell auf den Patienten beziehungsweise die patientenspezifische Operationsplanung angepasst sind.

## Claims

1. Trial repositioning block (1, 1', 1", 100) for defining at least one cutting plane on a femur F with a total knee arthroplasty, comprising :
- at least one patient-specific proximal reference region (10), which is placed against a distal surface of the femur,
- a distal sidewall (4, 4', 4"),
- and/or preferably a dorsal sidewall (5, 5', 5"),
**characterized in that**
- the distal sidewall (4, 4', 4") defines a distal reference surface (8, 8', 8"), which according to the patient-specific surgical plan coincides with the tibia cut (TS) with an extension of a knee joint,
- and/or the dorsal side wall (5, 5', 5") defines a dorsal reference surface (9, 9', 9"), which according to the patient-specific surgical plan coincides with the tibia cut (TS) with the flexion of the knee joint coincides with the tibia cut (TS),
wherein a thickness of the distal side wall (4, 4', 4") and/or the dorsal side wall (4, 4', 4") is individually adapted to the patient respectively to the patient-specific surgical plan.

2. Trial repositioning block (1) of claim 1, **characterized in that** the distal and/or the dorsal reference surface are formed directly by the surface of the respective sidewall.

3. Trial repositioning block (1) of claim 1, **characterized in that** the distal and/or the dorsal reference surface are formed by spacers (30), which are arranged on the sidewalls, wherein the spacers (30) preferably can be detachably fastened to the sidewalls.

4. Trial repositioning block (1) of either of claims 1 to 3, **characterized in that** the trial repositioning block further comprises an anterior (2) and a dorsal (3) block part, which can be fastened to one another and positioned detachably.

5. Trial repositioning block (1) of claim 4, **characterized in that** the anterior block part (2) comprises a cutting guide for a distal femur cut FSD or positioning means for a cutting block for at least one distal femur cut FSD, wherein the cutting guide or the positioning means define a reference plane for the distal femur cut FSD, which is aligned to the distal reference plane of the trial repositioning block such that these two planes correspond to an extension gap according to the patient-specific surgical plan.

6. Trial repositioning block (1) of claim 5, **characterized in that** the anterior sidewall is provided with correction positioning means, especially preferably in form the of correction pin borehole pairs or correction inserts, configured to distalize, proximalize, varisiate or valgisate the cutting guide for the first distal femur cut as needed.

7. Trial repositioning block (1) of claim 6, **characterized in that** the correction pin borehole pairs or correction inserts are provided for the following correction positions: +1, +2, +3, +4, 0, -1, -2, -3, -4; 1°, 2°, 3°, 4° varus/valgus in order to beable to distalize, proximalize, varisiate or valgisate a conventional distal cutting block or a cutting guide in the trial repositioning block as needed.

8. Trial repositioning block (1) of either of claims 4 to 7, **characterized in that** the distal block part further comprises positioning means, preferably in the form of pairs of pin holes for a cutting block or a cutting guide for a dorsal femur cut FSB and anterior femur cut FSA, which can guide a cutting guide/tool for producing the dorsal femur cut FSB and the anterior femur cut FSA, wherein the positioning means define the position of the cutting guide, which in turn defines a reference plane for the femur cut, which is aligned to the dorsal reference plane (9") of the trial repositioning block such that these two planes correspond to the flexion gap according to the patient-specific surgical plan.

9. Trial repositioning block (1) of claim 8, **characterized in that** the pairs of pin holes are provided for the following correction positions: 0°-7° inner and outer rotation in 1 ° steps and +/-1-4 mm anterior/posterior shift.

10. Trial repositioning block (1) of either of claims 5 to 9, **characterized in that** the anterior block part (2) comprises a cutting guide for an anterior femur cut FSA and/or a posterior femur cut and/or further femur cuts.

11. Trial repositioning block (1) of either of claims 4 to 10, **characterized in that** the dorsal block part (3) on the dorsal sidewall (5) and the anterior block part (2) on the distal sidewall (4) are provided with cut-outs to accommodate sensors and/or are provided with sensors.

12. Trial repositioning block (1) of either of the preceding claims, **characterized in that** at least the patient-specific proximal reference region (10) and preferably the dorsal and the distal sidewalls (3, 4) of the dorsal and anterior block parts are produced via a rapid prototyping method.

13. Trial repositioning block (1) of either of the preceding claims, **characterized in that** the dorsal block part can be detachably connected to one another by means of positive and/or force fitting connection means and can be positioned in a defined relative position to one another.

14. Method for producing a patient-specific trial repositioning block (1, 1', 1", 100) for defining at least one cutting plane on a femur F with a total knee arthroplasty, wherein
- based on 3D-data of the knee to be treated, at least one patient-specific proximal reference region (10) is produced which is placed against a distal surface of the femur F,
**characterized in that**
- a distal sidewall (4, 4', 4") is produced such that it defines a distal reference surface (8, 8', 8") which according to a patient-specific surgical plan with an extension of a knee joint coincides with a tibia cut (TS)
- and/or preferably a dorsal sidewall (5, 5', 5") is produced, which defines a dorsal reference surface (9, 9', 9"), which according to the patient-specific surgical plan with a flexion of the knee joint coincides with the tibia cut TS,
wherein a thickness of the distal side wall (4, 4', 4") and/or the dorsal side wall (4, 4', 4") is individually adapted to the patient respectively to the patient-specific surgical plan.

## Revendications

1. Bloc (1, 1', 1", 100) de repositionnement de sonde servant à définir au moins un plan de coupe sur un fémur F lors d'une arthroplastie totale du genou, comprenant :
au moins une partie proximale de référence (10) spécifique au patient, placée contre la surface distale du fémur F,
une paroi latérale distale (4, 4', 4") et/ou au moins une paroi latérale dorsale (5, 5', 5")
**caractérisé en ce que**
la paroi latérale distale (4, 4', 4") définit une surface distale de référence (8, 8', 8") qui dans la planification opératoire spécifique au patient coïncide avec la coupe de tibia TS lors de l'extension de l'articulation du genou et/ou
**en ce que** la paroi latérale dorsale (5, 5', 5") définit une surface dorsale de référence (9, 9', 9'') qui dans la planification opératoire spécifique au patient coïncide avec la coupe de tibia TS lors de la flexion de l'articulation du genou,
l'épaisseur de la paroi latérale distale (4, 4', 4") et/ou de la paroi latérale dorsale (5, 5', 5") étant adaptée individuellement au patient ou à la planification opératoire spécifique au patient.

2. Bloc (1) de repositionnement de sonde selon la revendication 1, **caractérisé en ce que** la surface distale de référence et/ou la surface dorsale de référence sont formées directement par la surface de la paroi latérale concernée.

3. Bloc (1) de repositionnement de sonde selon la revendication 1, **caractérisé en ce que** la surface distale de référence et/ou la surface dorsale de référence sont formées par des écarteurs (30) disposés sur les parois latérales, les écarteurs (30) pouvant être fixés de préférence de manière libérable sur les parois latérales.

4. Bloc (1) de repositionnement de sonde selon l'une des revendications 1 à 3, **caractérisé en ce que** le bloc de repositionnement de sonde comporte une partie antérieure (2) et une partie dorsale (3) de bloc qui peuvent être fixées et positionnées l'une par rapport à l'autre de manière libérable.

5. Bloc (1) de repositionnement de sonde selon la revendication 4, **caractérisé en ce que** la partie antérieure de bloc (2) comporte un guide de coupe d'une coupe distale de fémur FSD ou des moyens de positionnement d'un bloc de coupe d'au moins une coupe distale de fémur FSD, le guide de coupe ou les moyens de positionnement définissant un plan de référence de la coupe distale de fémur FSD orienté par rapport au plan distal de référence du bloc de repositionnement de sonde de telle sorte que ces deux plans correspondent à l'interstice d'extension de la planification opératoire spécifique au patient.

6. Bloc (1) de repositionnement de sonde selon la revendication 5, **caractérisé en ce que** la paroi latérale antérieure est dotée de moyens de positionnement de correction qui présentent de façon particulièrement préférable la forme de paires de trous et de goujons de correction ou de garnitures de correction pour pouvoir si nécessaire distaliser, proximaliser, variser ou valgiser le guide de coupe de la première coupe distale de fémur FSD.

7. Bloc (1) de repositionnement de sonde selon la revendication 6, **caractérisé en ce que** les moyens de positionnement sont prévus pour les positions de correction suivantes : +1, +2, +3, +4, 0, -1, -2, -3, - 4; 1°, 2°, 3°, 4° varus/valgus autour d'un bloc de coupe distal classique ou un guide de coupe dans le bloc de repositionnement de sonde, si nécessaire pour pouvoir distaliser, proximaliser, variser ou valgiser.

8. Bloc (1) de repositionnement de sonde selon l'une des revendications 5 à 7, **caractérisé en ce que** la partie distale de bloc est dotée de moyens de positionnement qui présentent de préférence la forme de paires de trous et goujons pour un bloc de coupe ou un guide de coupe d'une coupe dorsale de fémur FSB et d'une coupe antérieure de fémur FSA qui permet de guider un guide de coupe ou outil de coupe en vue d'établir la coupe dorsale de fémur FSB et la coupe antérieure de fémur FSA, les moyens de positionnement définissant la position du guide de coupe qui elle-même définit un plan de référence de la coupe de fémur qui est orienté par rapport au plan dorsal de référence (9") du bloc de repositionnement de sonde de telle sorte que ces deux plans correspondent à l'interstice de flexion de la planification opératoire spécifique au patient.

9. Bloc (1) de repositionnement de sonde selon la revendication 8, **caractérisé en ce que** des moyens de positionnement de correction sont prévus pour les positions de correction suivantes : 0°-7° en rotation intérieure et extérieure par pas de 1° et +/- 1-4 mm de dérive antérieure/postérieure.

10. Bloc (1) de repositionnement de sonde selon l'une des revendications 5 à 9, **caractérisé en ce que** la partie antérieure de bloc (2) comporte un guide de coupe pour une coupe antérieure de fémur FSA et/ou une coupe postérieure de fémur FSB et/ou d'autres coupes de fémur.

11. Bloc (1) de repositionnement de sonde selon l'une des revendications 4 à 10, **caractérisé en ce que** la partie dorsale de bloc (3) est dotée sur la paroi dorsale latérale (5) et la partie antérieure de bloc (2) sur la paroi latérale distale (4) de découpes de réception de capteurs et/ou de capteurs.

12. Bloc (1, 100) selon l'une des revendications précédentes, **caractérisé en ce qu'**au moins la partie proximale de référence (10) spécifique au patient et de préférence les parois latérales dorsales et les parois latérales distales (3, 4) des parties dorsales et antérieures de bloc sont réalisées par une opération de prototypage rapide.

13. Bloc (1, 100) selon l'une des revendications précédentes, **caractérisé en ce que** les parties dorsales de bloc peuvent être reliées de manière libérable l'une et l'autre par des moyens de correspondance géométrique et/ou de correspondance mécanique (12, 60, 61) et peuvent être placées les unes par rapport aux autres en des positions relatives définies.

14. Procédé de fabrication d'un bloc (1, 1', 1'', 100) de repositionnement de sonde spécifique au patient en vue de définir au moins un plan de coupe sur un fémur F lors d'une arthroplastie totale du genou, dans lequel
au moins une partie proximale de référence (10) spécifique au patient placée contre la surface distale du fémur F est réalisée sur la base de données 3D du genou à traiter,
**caractérisé en ce que**
une paroi latérale distale (4, 4', 4") est réalisée de manière à définir une surface distale de référence (8, 8', 8") qui dans la planification opératoire spécifique au patient coïncide avec la coupe de tibia (TS) lors de l'extension de l'articulation du genou et/ou
**en ce qu'**une paroi latérale dorsale (5, 5', 5") est réalisée et définit une surface dorsale de référence (9, 9', 9") qui dans la planification opératoire spécifique du patient coïncide avec la coupe de tibia TS lors de la flexion de l'articulation du genou,
l'épaisseur de la paroi latérale distale (4, 4', 4") et/ou de la paroi latérale dorsale (5, 5', 5'') étant adaptée individuellement au patient ou à la planification opératoire spécifique au patient.
